(19) **Europäisches Patentamt**
**European Patent Office**
**Office européen des brevets**

(11) **EP 4 579 582 A1**

(12) **EUROPEAN PATENT APPLICATION**

(43) Date of publication:
**02.07.2025 Bulletin 2025/27**

(21) Application number: **23220765.4**

(22) Date of filing: **29.12.2023**

(51) International Patent Classification (IPC):
*G06T 7/00* (2017.01)

(52) Cooperative Patent Classification (CPC):
**G06T 7/0012;** G06T 2207/10132;
G06T 2207/20081; G06T 2207/20084;
G06T 2207/30096

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Intelligyn AB**
**129 35 Hägersten (SE)**

(72) Inventors:
• **SMITH, Kevin**
**191 44 Sollentuna (SE)**

• **EPSTEIN, Elisabeth**
**129 35 Hägersten (SE)**
• **KONUK, Emir**
**169 41 Solna (SE)**
• **CHRISTIANSEN, Filip**
**112 30 Stockholm (SE)**
• **HERMAN, Pawel**
**184 94 Åkersberga (SE)**

(74) Representative: **Høiberg P/S**
**Adelgade 12**
**1304 Copenhagen K (DK)**

(54) **METHOD AND SYSTEM FOR A COMPUTER-AIDED DETECTION SYSTEM TO IMPROVE GENERALIZATION AND PROTECT AGAINST BIAS**

(57)     The invention regards a method for training a multi-class neural network for diagnosing ovarian tumors using ultrasound images, the method comprising obtaining ultrasound image data from a plurality of diagnostic centers, annotating each image of the image data, training a series of initial neural networks to perform multi-class classification, evaluating the performance of each initial neural network of the series of initial neural networks; identifying an optimal set of hyperparameter; and training a final neural network using the optimal set of hyperparameters.

EP 4 579 582 A1

## EP 4 579 582 A1

## Description

## Background

Ovarian cancer, distinguished by the highest mortality rate among gynecologic cancers, underscores the urgent need for advanced screening and diagnostic solutions. Despite the potential for a 90% survival rate in stage I cases, this achievement is realized in only 15% of instances, emphasizing the critical gaps in current diagnostic methodologies. Traditional screening approaches, proven effective in cervical cancer, face formidable challenges in the context of ovarian cancer due to its low prevalence in the general population.

Current diagnostic strategies include the annual serum CA125 glycoprotein antigen testing combined with transvaginal ultrasound (TVS). This offers a viable approach for individuals at high risk. However, these strategies prove impractical for the broader population, mainly due to the dual constraints of low prevalence and prohibitive costs. The limitations of CA125, characterized by a lack of specificity and potential false positives, intensify the need for a more robust and reliable diagnostic paradigm.

Based on this, ovarian lesion diagnosis relying on ultrasound examination is the most promising modality. Further, Computer-Aided Diagnosis has been applied to ovarian tumor detection and diagnosis. Machine learning algorithms have shown to excel at recognizing patterns within vast datasets. Additionally, machine learning models can integrate information from various sources, including imaging data, clinical history, and biomarker levels, providing a holistic view for more accurate discrimination between malignant and benign lesions.

However, the effectiveness of machine learning models is contingent upon various factors, notably the quality and representativeness of the training data. Current models often rely on datasets that are not representative of the diversity of the patient population. Additionally, the utilization of correct training techniques is imperative to craft a model that not only excels in specific scenarios but effectively generalizes across a spectrum of clinical contexts.

## Summary

The present inventors have realized that the variability in patient populations, imaging devices, examination protocols, and the skill levels of technicians often leads to issues with the generalization of diagnostic models across different clinical environments.

For instance, CAD systems developed and trained in one geographic region or with a single type of imaging device may not perform effectively when applied to different populations or with images captured from different ultrasound equipment. This lack of generalization can lead to overly-optimistic expectations and inconsistent diagnostic performance when deployed in a new setting.

The present inventors have further identified that biases can arise from various sources, including different frequencies of certain diagnoses, variable image quality, and the presence of text, doppler, or other artifacts in ultrasound images. These biases can affect CAD systems, significantly skewing the training of the machine learning models they depend on, leading to reduced accuracy and reliability. For instance, a model trained predominantly on high-quality images may perform poorly on lower-quality images, common in less-equipped medical facilities.

In view of the challenges of the prior art, there exists a need for a more robust machine learning model for aiding in diagnosing ovarian tumors using ultrasound images.

Thus, the present disclosure relates to a method for training a multi-class neural network for aiding in diagnosing ovarian tumors using ultrasound images, the method comprising:

a. obtaining ultrasound image data from a plurality of diagnostic centers, each center providing images of both malignant and benign ovarian tumors, the image data comprising a plurality of sets, wherein each set comprises image data of an ultrasound examination;
b. annotating each image of the image data, comprising labeling the image data with histological diagnoses organized into a hierarchical ontology;
c. training a series of initial neural networks to perform multi-class classification wherein said training comprises a leave-one-out cross validation, wherein, for each round of said cross validation, a different center is used as hold-out test data, and a new initial neural network is trained using training data that is derived from the remaining image data;
d. evaluating the performance of each initial neural network of the series of initial neural networks;
e. identifying an optimal set of hyperparameters that yield the most consistent performance across all hold-out centers, based on the performance and the hyperparameters of each initial neural network; and
f. training a final neural network using the optimal set of hyperparameters.

The method allows for training a more robust neural network for aiding in diagnosing ovarian tumors using ultrasound images.

The trained multi-class neural network is robust against real-world variances in ultrasound imaging data. The present disclosure relies on the use of image data of ultrasound examinations from a plurality of centers, and the multi-class neural network is trained in such a way that the model is generalized and prevents bias when deployed at different centers.

The use of training data that has been annotated with a histological diagnosis, and the structuring of said data into a detailed hierarchical ontology describing the diagnosis at various levels of granularity, in combination with the use of a leave-one-out cross-validation wherein each of a plurality of centers are used as the holdout data, in combination with a hierarchical sampling scheme along with a multi-class focal loss has been shown to result in a synergistic effect and offers a significantly improved neural network model for aiding in diagnosing ovarian tumors using ultrasound images. This synergistic combination of features ensures that biases that could otherwise skew the system's diagnostic accuracy are mitigated.

The leave-one-out cross-validation scheme is used to select hyperparameters proven to yield the most robust models when tested on held-out data. For each center, data from that center is isolated as a hold-out test set and a model is trained with data from the remaining centers, using hierarchical sampling and multi-class focal loss, resulting in the identification of optimized hyperparameters that demonstrate the most consistent and robust performance across all hold-out centers. After identification of the optimized hyperparameters, the final model may be trained using all image data and will have enhanced generalization capabilities.

In a further aspect, the present disclosure relates to method of determining a malignancy score of an ovarian tumor, the method comprising:

a. obtaining a set of ultrasound images of the ovarian tumor;

b. providing the set of ultrasound images to a trained multi-class neural network that has been trained according to the method as disclosed elsewhere herein, thereby determining the malignancy score of the ovarian tumor

In yet a further aspect, the present disclosure relates to a computer program product comprising instructions which, when the program is executed by a computer, cause the computer to carry out the method of determining a malignancy score of an ovarian tumor as disclosed elsewhere herein.

In yet a further aspect, the present disclosure relates to computer-aided detection (CAD) system for diagnosing an ovarian tumor of a patient using ultrasound image data of said patient, the system comprising:

- a data reception module arranged to receive the ultrasound image data;
- a lesion diagnosis module comprising a computer-readable medium having stored thereon a multi-class neural network for diagnosing ovarian tumors using ultrasound images;
- a malignancy scoring model, configured to calculate a malignancy score of the ultrasound image data.

Thus, following a training process, the CAD system employs a resulting robust neural network to calculate a malignancy score for each case. This score can for example be derived by obtaining probability estimates for each diagnostic class from the neural network, which processes images using the exponential moving average (EMA) of its weights for robustness. The system can then compute the risk of malignancy for each case by aggregating the probabilities associated with malignant sub-diagnoses and averaging the scores from all relevant case images. A decision rule can be applied where a case is predicted as malignant or benign based on whether this malignancy score exceeds a predetermined operating point. This process, along with the robustness and generalization capabilities of the neural network, ensures highly accurate and reliable ovarian tumor diagnosis from ultrasound imaging when deployed in a new setting.

**Description of drawings**

**Fig. 1** shows a protocol sequence diagram of a data curation process, according to an embodiment of the present disclosure.

**Fig. 2** shows a diagram of a hierarchical diagnostic ontology, according to an embodiment of the present disclosure.

**Fig. 3** shows a flowchart of a method of training a multi-class neural network, according to an embodiment of the present disclosure.

**Fig. 4** illustrates a process for calculating a malignancy score for a case, according to an embodiment of the present disclosure.

**Fig. 5** shows a flowchart of a computer aided detection (CAD) system, according to an embodiment of the present disclosure.

**Fig. 6** shows Q-Q plots of the differences in paired F1 scores between a multi-class neural network, trained according to an embodiment of the present disclosure, and expert examiners.

**Fig. 7** shows a comparison of individual human examiners and a multi-class neural network, trained according to an embodiment of the present disclosure.

**Fig. 8** shows a comparison of individual human examiners and a multi-class neural network, trained according to an embodiment of the present disclosure.

**Fig. 9** shows a comparison of AI models, trained according to an embodiment of the present disclosure, and expert and non-expert examiners.

**Fig. 10** shows a comparison of AI models, trained according to an embodiment of the present disclosure, and expert and non-expert examiners.

**Fig. 11** shows a comparison of AI models, trained according to an embodiment of the present disclosure, and expert and non-expert examiners.

**Fig. 12** shows F1 scores for expert and non-expert examiners vs a model, trained according to an embodiment of the present disclosure.

**Detailed description**

Terminology

The term "algorithm" as used herein, encompasses logic, hardware, firmware, software, or a combination thereof, configured to execute one or more functions as described explicitly in the present disclosure or as discernible by those skilled in the art based on the provided description. Such logic may include circuitry that possesses data processing and/or storage capabilities. Examples of such circuitry encompass, but are not restricted to, a microprocessor, one or more processors (such as processor cores), a programmable gate array, a microcontroller, an application-specific integrated circuit, wireless receiver, transmitter, and/or transceiver circuitry, semiconductor memory, or combinatorial logic.

The term "logic", as used herein, denotes computer code and/or instructions in the form of one or more software modules, such as executable code in various forms like executable applications, application programming interfaces (APIs), subroutines, functions, procedures, applets, servlets, routines, source code, object code, shared libraries/dynamic load libraries, or sets of instructions. These software modules may be stored in a suitable non-transitory storage medium or a transitory storage medium, including but not limited to electrical, optical, acoustical, or other forms of propagated signals such as carrier waves, infrared signals, or digital signals. Non-transitory storage medium examples include programmable circuits, semiconductor memory, non-persistent storage like volatile memory (e.g., various types of random access memory "RAM"), persistent storage like non-volatile memory (e.g., read-only memory "ROM," power-backed RAM, flash memory, phase-change memory, etc.), solid-state drives, hard disk drives, optical disc drives, or portable memory devices. When used as firmware, the executable code is stored in persistent storage.

The term "user", as used herein, represents an individual utilizing or controlling the system and/or method of the present disclosure. In this context, the user may encompass clinicians, diagnosticians, doctors, technicians, students, controllers, and administrators, the terms of which are used interchangeably through the application. The terms "identified," "processed," and "selected", as used herein, are used interchangeably, regardless of tense, to signify an automated computerized process executed by the system in one or more processes employing at least one processor.

The terms "model," "network," and "neural network", as used herein, are used interchangeably to represent the computational structure within the system that processes data and makes predictions or classifications. This structure, typically consisting of interconnected layers and nodes, utilizes algorithms and learns from data to perform specific tasks related to ovarian tumor detection in ultrasound images. The model, network, or neural network may include various architectures, such as convolutional neural networks (CNNs), and is integral to the system's ability to analyze, interpret, and classify medical imaging data. Its functionality is central to the Computer-Aided Detection (CAD) system and encompasses the execution of processes like image analysis, feature extraction, and diagnostic classification. These terms are used irrespective of the specific configuration or complexity of the underlying architecture and are fundamental to the operation and effectiveness of the present disclosure.

The term "client", as used herein, refers to any software program that connects to a CAD lesion application.

The term "server", as used herein, typically denotes a CAD lesion application that listens for one or more clients unless otherwise specified.

The term "post-processing", as used herein, signifies one or more algorithms applied to an inputted ultrasound image.

The term "computerized", as used herein, generally indicates that corresponding operations are performed by hardware in conjunction with software and/or firmware.

The term hyperparameters, as used herein, are external configuration settings that are not learned from the data but are set prior to the training process. These parameters are typically not intrinsic to the model but influence the learning process and the structure of the trained model. As such, hyperparameters are typically distinct from model parameters, which are learned during training, such as network weights.

The acronym "PACS" represents Picture Archival and Communication System.

The acronym "DICOM" stands for Digital Imaging and Communications in Medicine.

The acronym "UI" represents User Interface.

The acronym "PHI" refers to Private Health Information.

The acronym "EHR" refers to Electronic Health Records.

The acronym "ROI" refers to a Region of Interest.

The term "computerized" generally indicates that corresponding operations are performed by hardware in conjunction with software and/or firmware.

The term hierarchical diagnostic ontology refers to a structured framework that organizes and represents knowledge about diagnoses and medical conditions in a hierarchical manner. This ontology is designed to capture relationships between different diagnostic entities, arranging them in a tree-like structure that reflects their hierarchical and often taxonomic relationships. Ontologies are knowledge representation models that define concepts and the relationships between them in a specific domain.

The inventors of the present application have discerned effective solutions to overcome the shortcomings identified in prior art methods and systems.

In view of this, the present disclosure relates, in a first aspect, to a method for training a multi-class neural network. The neural network may for example be arranged to aid in diagnosing ovarian tumors using ultrasound images. As such, the neural network may form part, or be interpreted as, a decision support system.

The method of the present disclosure may comprise a plurality of steps that results in the generation of a trained multi-class neural network, according to an embodiment of the present disclosure.

In one embodiment, the method comprises a step of obtaining ultrasound image data. The image data may for example be obtained from a plurality of centers. Said center may for example be a diagnostic center, or another type of medical facility. The center may for example be a diagnostic imaging center, also known as a medical diagnostic center, and may be a facility dedicated to performing various diagnostic tests and medical imaging procedures to assist in the diagnosis and evaluation of health conditions. These centers can be equipped with advanced medical imaging technologies and diagnostic equipment, such as X-ray machines, ultrasound devices, CT scanners, MRI machines, and other specialized tools.

Further, the center may alternatively or additionally be a primary care center, a laboratory, a women's health center, a specialized clinic and/or an urgent care center.

In one embodiment of the present disclosure, each center provides images of both malignant and benign ovarian tumors. However, in other embodiments this is not a requirement. The image data can comprise a plurality of sets, wherein each set may have been obtained by a different ultrasound examination. As such, each set may comprise image data of an ultrasound examination of an individual patient.

In one embodiment of the present disclosure, the method comprises a step of annotating, while other embodiments do not comprise such a step. Said step may comprise annotating images of the image data. For example, each image of the image data. The step of annotating may for example comprise labeling the image data. The labeling may be based on expert review of the image data, and the labeling may therefore be referred to as expert-labeling. The labeling may for example comprise labeling with histological diagnoses organized into a hierarchical ontology. The hierarchical ontology, also referred herein as hierarchical diagnostic ontology, can be a predefined ontology. For embodiments of the present disclosure not comprising a step of annotating, the image data may have, prior to being obtained, have been annotated.

Thus, each set may correspond to an ultrasound examination of an individual patient. Each set may be assessed by an expert, who assigns a categorical label of the ontology, for example for each granularity of the ontology or from the most or least granularity of the ontology. This allows for a detailed and systematic annotation for training the neural network to recognize and classify a wide range of ovarian tumor types.

In one embodiment of the present disclosure, the method comprises a step of training a series of initial neural networks. The training of the initial neural network may be carried out in order to identify optimized parameters, before training a final neural network. The training of the initial neural network may for example be arranged such that the initial neural network can perform multi-class classification, thereby increasing the performance of the neural network.

The training may for example comprise a leave-one-out cross validation, such as a special variant of a leave-one-out cross validation approach.

"Leave-One-Out Cross-Validation" (LOOCV) is typically used to assess the performance of a machine learning model by systematically leaving out one data point as the validation set and training the model on the remaining data. This process is repeated for each data point, and the performance is evaluated based on the average performance across all iterations. LOOCV is particularly useful when the dataset is limited in size.

In the presently disclosed embodiment, the LOOCV can be implemented in such a way that, for each round of said cross validation, a different center is used as hold-out test data. Additionally, a new initial neural network can be trained using training data that is derived from the remaining image data. The training data may therefore be different for each round of the LOOCV. Further, the training data may be hierarchically sampled, such as for each round of the LOOCV and/or for each training round of each initial neural network.

In one embodiment of the present disclosure, the method comprises a step of evaluating the performance of each initial neural network of the series of initial neural networks. The initial neural networks may be evaluated based on any suitable performance metrics, in order to reflect any type of property of the initial neural networks. For example, the initial neural networks may be evaluated based on what hyperparameter(s) that yield the most consistent performance across all hold-out centers. Alternatively, the neural networks may be evaluated based on what set of hyperparameter(s) that has the best performance, such as which yield the most consistent performance across all hold-out centers. A set of hyperparameters may correspond to the hyperparameters of a plurality of the initial neural networks, i.e. the hyperparameters that were used to train the initial neural networks.

In one embodiment of the present disclosure, the method comprises a step of identifying an optimal set of hyperparameters, for example the set of hyperparameters that have the best performance. The step may for example be arranged to identify the optimal set of hyperparameters that yield the most consistent performance across all hold-out centers, and/or good performance and consistent performance.

The step may be arranged to identify the optimal set of hyperparameters based on the performance and the hyperparameters of each initial neural network. As such, the optimal set of hyperparameters may be identified based on the performance of each initial neural network. The identification may alternatively or additionally comprise a mathematical step wherein the hyperparameters of the initial neural networks (i.e. the hyperparameters of the best performing initial neural network, or the set of hyperparameters of the best performing plurality of networks) are processed, in order to derive the optimal set of hyperparameters.

In one embodiment of the present disclosure, the method comprises a step of training a final neural network using the optimal set of hyperparameters. Thus, the final neural network may be trained using an optimal set of hyperparameters identified based on the evaluation of the plurality of initial neural networks.

Image data

In one embodiment of the present disclosure, each set comprises a plurality of images, such as grayscale and/or doppler images.

The image data may thus include both grayscale and doppler ultrasound images. The image data may for example be provided in a DICOM or JPEG format. The image data may be arranged to ensure a comprehensive dataset that reflects the diversity of imaging techniques used in clinical practice.

In one embodiment of the present disclosure, each set comprises a mixture of grayscale and doppler images.

Thus, in one embodiment of the present disclosure, each set of the image data may comprise at least one doppler image. In this way, varied imaging modalities are incorporated into the training phase.

In one embodiment of the present disclosure, the centers are located in different cities and/or countries. Further, as disclosed elsewhere herein, the center may be different types of healthcare facilities, such as a diagnostic imaging center, a primary care center, a laboratory, a women's health center, a specialized clinic and/or an urgent care center. In one embodiment of the present disclosure, the center is a diagnostic imaging center, such as a diagnostic ultrasound imaging center.

In one embodiment of the present disclosure, the image data from each center has been generated by a different combination of ultrasound examiner, ultrasound system, imaging modality and/or the presence of calipers. Thus, the image data of each center may have been generated in a different way. This may be a reason for the introduction of bias, in case a conventional training strategy is used to train a neural network. However, the presently disclosed method of training a multi-class neural network, is arranged in such a way as to prevent bias in the final trained model and for generalization of said model.

It should also be noted that, even in the event that the plurality of centers have been set up in a way so as to generate data in an identical way, the present inventors have realized that the resulting imaging data will anyway have subtle differences that would result in the introduction of bias in a final trained model, in case the final model would have been trained in a conventional way. Thus, only, the presently claimed method of training a multi-class neural network is capable of realizing a final trained neural network that is generalized and wherein bias is prevented. As such, the exact differences between the centers may be of limited relevance for the presently claimed embodiment, as the centers may have even been set up in a way so as to eliminate any differences, while still introducing bias(es). Thus, even in the event that the centers have been set up to generate image data in an identical way, it has been shown that the resulting image data can comprise features that result in bias when a neural network is trained in a conventional way.

Therefore, the presently claimed method of training a multi-class neural network is not limited to a particular type of differences between centers, as the multi-class network is capable of training a final multi-class neural network in a way that prevents bias irrespective of the image data and/or the differences between the centers.

In one embodiment of the present disclosure, the image data is provided in a DICOM or a JPEG format. Thus, the image data may be provided in DICOM (Digital Imaging and Communications in Medicine) and/or JPEG (Joint Photographic Experts Group) format. Alternatively, or additionally, the image data may be provided in PNG (Portable Network Graphics), TIFF (Tagged Image File Format), RAW (Uncompressed), NIfTI (Neuroimaging Informatics Technology Initiative), HDF5 (Hierarchical Data Format version 5) and/or JPEG2000.

In one embodiment of the present disclosure, the image data of each center, comprises both malignant and benign cases. The ratio between the malignant and benign cases can be any value.

In one embodiment of the present disclosure, the image data of each center (and or the set of images of each center) has a ratio of malignant and benign cases in the range between 30%-70%:30%-70%, such as 45%-55%:45%-55%.

Thus, for example 30%-70% of the image data (and/or the sets of image data) of each center may be malignant cases, while the remaining image data (i.e. the remaining 30-70%) of that center is benign cases. In general, the ratio may be P:1-P, wherein P may be from 30% to 70 %, such as from 35% to 65%, such as from 40% to 60%, such as from 45% to 65%, wherein P is the ratio of malignant cases of the image data and/or the ratio of malignant sets of the image data.

In embodiments wherein the image data of a center has a significant deviation from an equal balance between malignant benign cases, such as a set of image data, the image data may be adjusted to compensate for this. For example, the image data may be sampled such that the image data of that center, such as the set of image data of that center has a substantially equal balance between malignant and benign cases, such wherein the ratio between malignant and benign cases may be P:1-P, wherein P may be from 30% to 70 %, such as from 35% to 65%, such as from 40% to 60%, such as from 45% to 65%, wherein P is the ratio of malignant cases of the image data and/or the ratio of malignant sets of the image data.

In one embodiment of the present disclosure, the image data of each center has been obtained by a plurality of examinations of patients, wherein said examinations have taken place within an acquisition period of 6 months, such as within 4 months, such as within 2 months. Malignant and benign cases can appear approximately uniformly throughout the acquisition period.

In one embodiment of the present disclosure, the interval between an ultrasound examination of a patient whereby image data is obtained, and surgery of that individual patient, was less than 6 months.

In one embodiment of the present disclosure, the image data has been generated by a plurality of ultrasound examiners, a plurality of ultrasound systems, a plurality of imaging modalities and/or with the presence and absence of calipers (i.e. calipers in the obtained image).

The obtained image data can encompass images captured from a wide range of ultrasound device types and models. This diversity in device types can further enhance the robustness of the model and make it generalizable across different technological platforms used in various medical centers.

Annotating

In one embodiment of the present disclosure, the step of annotating comprises annotating each image of the image data with a region-of-interest (ROI).

In one embodiment of the present disclosure, the step of annotating comprises the use of an artificial intelligence (AI) detector, such as for annotating each image of the image data with a region-of-interest (ROI).

In one embodiment of the present disclosure, the step of annotating comprises a step of labeling, such as expert labeling. The step of labeling may for example be subsequent to a step of annotating each image of the image data with a region-of-interest (ROI).

In one embodiment of the present disclosure, the step of labeling (e.g. expert annotation) may comprise labeling the images with histological diagnoses that correspond to a pre-defined hierarchical ontology.

The hierarchical ontology can provide a structured framework for labeling, encompassing various levels of diagnostic detail from a broad categorization (such as malignant or benign) to specific sub-diagnoses.

Each exam (i.e. set of image data) can be assessed by an expert, who assigns a categorical label to the set, such as from the most granular level of the ontology, from the least granular level of ontology, and/or from each granular level of the ontology. In a specific example, the labeling is arranged such that the set is provided with a label from the most granular level of the ontology.

This detailed and systematic hierarchical annotation ensures, together with the other features of the present disclosure, that a robust and generalized neural network can be trained, that can classify a wide range of ovarian tumor types accurately.

In one embodiment of the present disclosure, the step of annotating comprises labeling each image of the image data with histological diagnoses that correspond to a pre-defined hierarchical ontology.

For example, the hierarchical ontology may be arranged such that, at the highest level, the ontology bifurcates diagnoses into the fundamental categories of benign and malignant, and may as such form the basis of the diagnostic process.

The hierarchical ontology may comprise one or more additional levels, that each have a plurality of categories.

In one embodiment of the present disclosure, the hierarchical ontology comprises a first level and a second level. In one embodiment of the present disclosure, the hierarchical ontology comprises a first level, a second level, and a third level. In one embodiment of the present disclosure, the hierarchical ontology comprises a first level, a second level, a third level, and a fourth level.

For example, the hierarchical ontology may comprise a second level that expands the binary classification into a plurality of categories, such as of ovarian conditions. For example ten distinct categories of ovarian conditions.

The third level may comprise a plurality of categories, such as 18 categories that offer a more intricate classification based on histological and morphological traits.

The fourth level may comprise forty-four categories. The fourth level may cover an extensive range of ovarian tumor subtypes and/or histological and/or morphological traits. For example:

BENIGN: Endometrioma, Dermoid, Simple cyst, Inclusion cyst, Paraovarian cyst, Functional cyst, Hydrosalpinx, Pyosalpinx / Tubo-ovarian abscess, Peritoneal cyst, Fibroma, Thecoma, Myoma, Cystadenoma (serous), Cystadenoma (mucinous), Cystadenofibroma (serous), Cystadenofibroma (mucinous), Struma ovarii, Brenner tumor, Endometrioma (decidualized), Schwannoma, Leydig cell tumor, Other rare benign.

MALIGNANT: Borderline (serous), Borderline (mucinous intestinal), Ovarian cancer (serous), Tubal cancer, Carcinosarcoma, Other malignancy (epithelial), Ovarian cancer (mucinous), Ovarian cancer (endometrioid), Ovarian cancer (clear-cell), Granulosa cell tumor, Yolc sac tumor, Dysgerminoma, Sertoli-Leydig cell tumor, Mixed Germcell tumor, Other malignancy (non-epithelial), Metastasis (colorectal), Metastasis (pancreas), Metastasis (gastric), Metastasis (breast), Metastasis (endometrial), Metastasis (lymphoma), Metastasis (other).

By the use of a hierarchical structure the set of image data (e.g. the individual patient cases) can be classified and/or annotated with precision, will also significantly boost the capability of the multi-class neural network, together with the other synergistic features of the present disclosure. Thus, the training may, in one embodiment be arranged to discern subtle differences across these detailed categories. As a result, the trained model may become proficient in accurately identifying benign and malignant cases.

In other examples of the present embodiment, the hierarchical ontology comprises a first level that comprises or consists of the classifications benign and malignant.

The hierarchical ontology may comprise a second level that comprises or consists of the classifications benign, borderline and malignant.

Borderline, as used herein, may refer to a type of local cancer, without the same risk of dissemination as true invasive cancer. In general, borderline can be an important group to separate, as the prognosis is better and fertility sparing surgery can be offered to borderline but typically not to cancer.

The hierarchical ontology may comprise a third level that comprises or consists of a plurality of classifications, for example include 10 classes (e.g. 5 benign and 5 malignant).

The hierarchical ontology may comprise a fourth level that comprises or consists of a plurality of classifications, for example 18 classes (10 benign and 8 malignant).

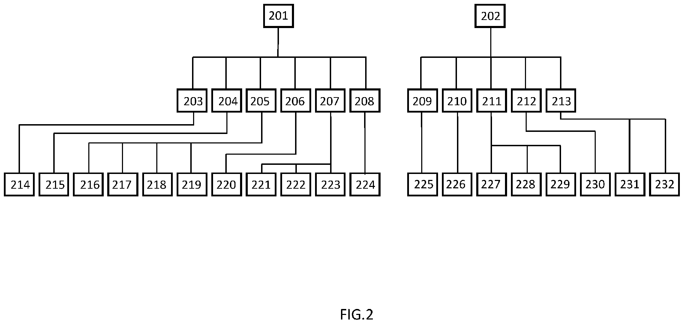

An example of a hierarchical ontology (also referred to herein as hierarchical diagnosis ontology) is shown in Fig. 2.

In one embodiment of the present disclosure, the hierarchical ontology comprises multiple hierarchical levels, such as a first level and a second level, and optionally a third level, and optionally a fourth level, and optionally further levels.

In one embodiment of the present disclosure, the step of annotating comprises artifact annotation, wherein artifacts, such as text or other annotations is removed, such as by setting the pixel values to 0. This annotation process can lay the groundwork for more detailed analysis and classification.

Preprocessing

In one embodiment of the present disclosure, the method further comprises a step of preprocessing the image data, before the step of training, such as after the step of annotating.

The preprocessing may comprise one or a plurality of steps that are arranged to standardize, enhance, and/or secure the images of the image data, typically before they are used to train a neural network, such as an initial multi-class network and/or final multi-class neural network.

The step of preprocessing may comprise a step to ensure that all images of the image data are compliant with privacy regulations. This may include the removal or anonymization of any patient-identifiable information and/or PHI present in the image data or associated metadata. This step can be crucial to uphold patient confidentiality and adhere to legal standards, such as GDPR or HIPAA, depending on the jurisdiction.

The preprocessing may be arranged to crop, each image, for example such as to focus on a defined Region of Interest (ROI). The ROI can for example contain the pertinent diagnostic features. As mentioned elsewhere herein, the ROI may be determined manually, semiautomatic, or automatic. For example, the ROI may be determined, for each image, through an automated detection algorithm or through expert annotation.

Cropping can be performed by selecting the pixel boundaries that encapsulate the ROI and removing the extraneous parts of the image.

Mathematically, if $(x1, y1)$ and $(x2, y2)$ represent the top-left and bottom-right coordinates of an ROI of an image, the cropped image I_cropped can be obtained by I_cropped = I [x1 : x2, y1: y2], where I is the original image.

In one embodiment of the present disclosure, the step of preprocessing may comprise a step of resizing. Said step of resizing may be arranged to resize the images of the image data to a standard dimension, for example to ensure uniformity. The resizing can be arranged to maintain the aspect ratio, and thereby prevent distortion of the image features.

In one example, I_standard denotes the resized image, and d_width x d_height be the standard dimensions, then: I_standard = resize(I_cropped, d_width, d_height)
where the resize function can be a standard interpolation function such as bicubic or nearest neighbor.

In one embodiment of the present disclosure, the step of preprocessing comprises a step of normalization. Said step of normalization may be arranged such that the pixel values of the images are normalized to ensure that they fall within a standard range, typically between 0 and 1.

This normalization can aid in the training of the neural network by providing a consistent scale for image intensities. If $p_{ij}$ represents the pixel value at position $(i, j)$ in I_standard and $p\_max$ and $p\_min$ represent the maximum and minimum pixel values in the original image, the normalized image I_normalized can be given by I_normalized $(i, j) = (p\_ij - p\_min) / (p\_max - p\_min)$.

The above-given example of a normalization equation is arranged such that each pixel value is scaled to a range between 0 and 1. These preprocessing steps not only standardizes the data but also enhances the relevant features, facilitating improved diagnostic accuracy.

In specific examples, the image data obtained from a center is preprocessed and selected, such as sampled, to fulfill a specific criteria. Thus, the image data may be sampled from a larger image set. In one example, the image data may be selected and/or sampled on the following criteria:

- The number of malignant and benign cases is approximately balanced, as mentioned elsewhere herein. If the natural occurrence at a center does not provide this balance, the sample can be adjusted to achieve it.
- The data from each center should be collected over a specific, consistent time window, as mentioned elsewhere herein. This ensures that both malignant and benign cases are uniformly represented throughout the acquisition period.
- the dataset from each center can include multiple images per exam. At least one of these images can be a doppler ultrasound image, to ensure the inclusion of varied imaging modalities.
- each center can contribute with images captured by multiple ultrasound device types, in this way the dataset can cover a broad spectrum of imaging technologies.

In one embodiment of the present disclosure, the preprocessing may comprise a quality assessment for each image, for example to ensure that the data used in training the model meets specific quality standards. Criteria for quality assessment can for example include image clarity, resolution, and the absence of obfuscating artifacts. If an image does not meet these quality standards, it is excluded from the dataset. This exclusion is necessary to maintain the integrity and reliability of the training process.

In one embodiment of the present disclosure, the preprocessing comprises one or more of anonymization, cropping, resizing, normalization of pixel values, and/or sampling of images. Each of said steps may be arranged as disclosed elsewhere herein.

Hierarchical ontology

In one embodiment of the present disclosure, the hierarchical ontology comprises a plurality of levels of granularity.

In one embodiment of the present disclosure, the hierarchical ontology comprises at least three levels of granularity, such as at least four levels of granularity.

In one embodiment of the present disclosure, the hierarchical ontology comprises a fourth level of ovarian tumor subtypes and/or histological and morphological traits, a third level of ovarian lesion classification, a second level of detailed classification, and a first level of binary classification.

In one embodiment of the present disclosure, the fourth level comprises at least 30 classifications, such as 44 classifications. In one embodiment of the present disclosure, the third level comprises at least 10 classifications, such as 18 classifications. In one embodiment of the present disclosure, the second level comprises at least 5 classifications, such as 10 classifications. In one embodiment of the present disclosure, the first level comprises 2 classifications, such as binary and malignant.

Initial training

The initial training may be an implementation of a leave-one-out cross-validation loop. In this loop, data from one center can be left out for validation purposes, while the remaining data can be used for training. This approach can be repeated N times (wherein N corresponds to the number of centers of the image data), each time data from a different center can be left out.

Hyperparameters for training the neural network can be chosen at the start of each training iteration. For example, the hyperparameters may comprise the learning rate, the learning schedule, and the batch size. Alternatively, the hyperparameters may comprise the learning rate, the learning schedule, and the batch size, the architecture of the network and the training length. Additional hyperparameters are known to the skilled person.

The hyperparameters can for example be chosen according to a grid search or a Bayesian Optimization. Network weights can be initialized either randomly or using pre-trained weights from models trained on natural scenes, such as ImageNet, from other medical imaging modalities e.g. x-ray, other medical domains, e.g. mammograms, or other ultrasound models.

In one example, the multi-class neural network is trained through several iterations. Each iteration may comprise a Hierarchical Data Sampling, a Forward Pass and a Loss Calculation, such as focal loss, and a Backpropagation. In other examples, the hierarchical data sampling generated a set that is also used for the subsequent iterations. However, hierarchical data sampling for each iteration has been shown to have a synergistic effect together with the other features of the present disclosure, such as the specific implementation of a LOOCV approach wherein each center is used as a holdout data while training a new neural network model. This results in a synergistic effect and generates a significantly more robust neural network that is generalized (i.e. bias has been prevented, and the model can thus be used to accurately assess image sets of different centers).

In one example, the hierarchical data sampling is implemented in the neural network training, with an emphasis on enriching under-represented categories, by balancing the dataset across the different image categories. Such as wherein the image categories comprises or consists of a plurality of: diagnosis type, imaging modality, device type, and the presence of calipers.

Thus, the categories may comprises one or more, such as all of: Diagnosis Type (e.g., malignant, benign), imaging modality (e.g., grayscale, doppler), device type (e.g., different ultrasound machine models), presence of calipers (binary: present or not present).

In one example of the hierarchical sampling, for each category, the frequency of each subcategory can be calculated by ($f(c_i)$ = Number of samples in $c_i$ / Total number of samples in C).

The sampling weights can then be determined by inverting these frequencies and multiplying them by a factor E to enrich under-represented image categories ($w(c_i) = E / f(c_i)$).

During training, batches can be formed, such as for each training iteration of an initial neural network, by randomly selecting samples according to these adjusted weights.

If B is a batch and N is the batch size, then batch B comprises N samples ($x_1, x_2, ..., x_N$), each chosen based on the weighted probability. This method ensures that each training batch is not only representative of the overall diversity in the dataset but also emphasizes the lesser-represented categories, enhancing the model's ability to learn from a broader range of cases. Furthermore, the combination of hierarchical sampling and the use of a LOOCV with each center used as a holdout set, wherein multiple initial neural networks are trained have been shown to offer a synergistic effect in terms of identifying the optimal set of hyperparameters for training a final robust, generalized multi-class neural network.

Following hierarchical sampling, the sampled image data (batch) may be passed through a multi-class neural network and the loss is calculated. In the forward pass of a batch through the neural network, each input image from the batch is processed by the network. This can involve the input data passing through multiple layers of the network, for example including convolutional layers, activation functions, and pooling layers.

Each layer can be arranged to transform the data by extracting and/or amplifying features relevant for tumor detection. The final layer can be arranged to output a set of probability scores for each diagnostic class in the hierarchical ontology, indicating the likelihood of the image belonging to each classification.

In one embodiment of the present disclosure, each initial neural network is trained using multi-class focal loss.

In one embodiment of the present disclosure, the multi-class focal loss is calculated for each iteration and for each image in the batch.

In one embodiment of the present disclosure, the training data is derived from the image data, for each round of the cross-validation, such as by hierarchical sampling. Thus, the hierarchical sampling may be arranged to obtain a new batch, for each training iteration of an initial neural network. In general, the hierarchical sampling is carried out on the training data of each round of the LOOCV, wherein different centers are used as the holdout set. Thus hierarchical sampling can also be arranged to sample a batch in processes wherein the respective holdout sets are excluded.

In one embodiment of the present disclosure, the image data is, for each round of the cross-validation, randomly split, according to a predetermined ratio, into a training set used for deriving the training data and a validation set used for selection of hyperparameters. Typically, wherein each round of the cross-validation has a different center as the holdout data.

In one embodiment of the present disclosure, the training comprises using a multi-class focal loss algorithm for calculating the loss for each image in the batch based on the output probabilities from a forward pass, such as for each training iteration of an initial and/or final multi-class neural network.

By the use of multi-class focal loss, the training can be focused on hard-to-classify examples. The multi-class focal loss can be mathematically defined as:

$$FL(p\_t) = -alpha * (1 - p\_t)\text{\textasciicircum}gamma * \log(p\_t)$$

where p_t is the probability of the correct class determined by the network, and alpha and gamma are tunable parameters. Alpha balances the importance given to each class, while gamma focuses the training on hard examples. The loss is summed or averaged over all images in the batch. Following the loss calculation, backpropagation can be used to update the network weights. In backpropagation, the focal loss calculated for each image can be used to determine how much each weight in the network contributed to the error. The network can then be arranged to adjust its weights to minimize this error. This can involve computing the gradient of the loss function with respect to each weight and adjusting the weights in the opposite direction of the gradient. The learning rate determines the size of these adjustments. This process iteratively improves the network's performance.

The focal loss is designed to address the class imbalance problem, which can occur in object detection tasks where the number of background (non-object) samples significantly outweighs the number of foreground (object) samples. The idea behind focal loss is that it down-weights the contribution of easy-to-classify samples and focuses more on the difficult examples, thus giving more emphasis to the misclassified or harder-to-classify samples. Multi-class Focal Loss is an extension of Focal Loss designed for tasks where there are multiple classes, and it helps improve the training of deep learning models for object detection in the presence of class imbalance.

Thus, the use of multi-class focal loss offers a synergistic effect in combination with the hierarchical ontology of the presently disclosed embodiment. In addition, multi-class focal loss offers a synergistic effect in combination with the LOOCV of the present disclosure, as it leads to a significantly more robust and generalized neural network for classifying ovarian lesions.

In one embodiment of the present disclosure, the LOOCV loop is arranged for training of multiple subsets of data. After said training with each subset of data multiple times to search for good hyperparameters, a statistical analysis of the hyperparameters can be conducted.

In one embodiment of the present disclosure, the method comprises a statistical analysis of the hyperparameters of the initial neural networks, such as in order to identify an optimal set of hyperparameters for training of a robust and generalized multi-class neural network.

The statistical analysis may be arranged such as to identify the set of hyperparameters that provide the highest average performance (measured in AUC) and the least variance across all validation sets. The most robust hyperparameters can for example be those that consistently (i.e. across the centers) yield high accuracy and generalizability.

Identification of an optimal set of hyperparameters may comprise choosing the set of hyperparameters that maximizes the average AUC while minimizing the performance variance. In one example, the step of identification comprises applying a mathematical algorithm to a set of hyperparameters, such as a set of top candidate hyperparameters, in order to obtain the optimal set of hyperparameters.

Evaluating

In one embodiment of the present disclosure, the step of evaluating comprises calculating, for each initial neural network, a performance metrics, such as the area under the received operating characteristics (AUC), such as for binary classification of each set. Thus, for each initial neural network, each holdout set may be used as a test set for each respective trained initial multi-class neural network.

As disclosed elsewhere herein, the evaluation may comprise performing a quantitative analysis of the performance of each initial multi-class neural network, for example in terms of the robustness and/or generalization of each neural network.

Identifying

In one embodiment of the present disclosure, the step of identifying comprises identifying the optimal set of hyperparameters that yields the highest average performance and least variance across all hold-out centers.

In one embodiment of the present disclosure, the step of identifying comprises identifying an optimal set of hyperparameters by performing a statistical analysis of the performance and the hyperparameters of each initial neural network.

Type of network

In one embodiment of the present disclosure, the initial and/or final neural network is a vision transformer, such as a data efficient image transformer.

A Vision Transformer (ViT) is a type of neural network architecture that, unlike traditional convolutional neural networks (CNNs) that rely on convolutional layers for feature extraction, use self-attention mechanisms to capture global context information from the input image. The architecture was introduced in the paper titled "Attention is All You Need" by Vaswani et al. in 2017, originally proposed for natural language processing tasks.

The Data-efficient Image Transformer (DeiT) is a specific instance or variant of the Vision Transformer architecture designed for image classification tasks. It was introduced in the paper titled "Training Vision Transformers on Noisy Large-Scale Data" by Touvron et al. in 2020.

In one embodiment of the present disclosure, the network weights of the initial neural networks and/or the final neural network are initialized either randomly, or by transfer learning, such as using pre-trained weights from models trained on natural scenes, such as ImageNet, from other medical imaging modalities e.g. x-ray, other medical domains, e.g. mammograms, or other ultrasound.

Final model

In one embodiment of the present disclosure, the final neural network is trained using the image data, such as the full image data.

In one example, the final multi-class neural network is trained similarly, such as identical, to the initial neural networks. The differences include, such as are limited to, the use of optimal set of hyperparameters and the complete image data, such as a complete balanced image data that has been pre-processed as disclosed elsewhere herein.

In one embodiment of the present disclosure, the final neural network is arranged for providing decision support for diagnosing ovarian cancer.

Once the optimal set of hyperparameters have been identified, the entire dataset may be pre-processed and/or used for the final model training. This training can be, in many ways, identical to the training of the initial neural networks. The training of the final model can also employ hierarchical data sampling and multi-class focal loss, as described elsewhere herein. The synergistic effect of these features allows, as mentioned elsewhere herein, for a synergistic effect as a surprisingly robust and generalized neural network model can be trained.

In examples wherein the entire dataset is used, the inclusion of the entire dataset in this stage can ensure that the final model is well-trained and capable of generalizing across diverse clinical scenarios. The outcome of this training process is a final model that is proven to be robust and generalized.

Further, the multi-class final neural network can be arranged to classify ovarian tumors in ultrasound images, accounting for variations in diagnosis types, imaging modalities, device types, and the presence of calipers.

Hierarchical sampling

In one embodiment of the present disclosure, the training data is derived from the image data by hierarchical sampling, such as for training each initial neural network and/or for training the final neural network. For training of each

**EP 4 579 582 A1**

initial neural network the image data of a different center is used as the holdout data, and may therefore be excluded from the training data.

In one embodiment of the present disclosure, the hierarchical sampling is arranged such that the training data comprises a balanced distribution of the categories of the image data.

In one embodiment of the present disclosure the balanced distribution of data is arranged such that the training data for training each initial neural network and/or final neural network comprises each category in terms of ovarian lesion classification, imaging modality, device type, and the presence of calipers.

In one embodiment of the present disclosure, the hierarchical sampling comprises use of a sampling weight for each category, wherein said sampling weight is determined by the inverse of the frequency of each category.

In one embodiment of the present disclosure the sampling weights are determined by the inverse of the frequency of each category multiplied by a factor E, thereby enriching under-represented categories of the image data.

In a further aspect, the present disclosure relates to use of a trained multi-class neural network for diagnosing ovarian tumors using ultrasound images.

Thus, ultrasound images may be provided to the trained multi-class neural network, and the network may be arranged to provide an output that can aid in the diagnosis of ovarian tumors.

In one embodiment of the present disclosure, the multi-class neural network has been trained according to the method of training a multi-class neural network for aiding in diagnosing ovarian tumors using ultrasound images.

In a further aspect, the present disclosure relates to a method of determining a malignancy score of an ovarian tumor. The method may for example comprise obtaining a set of ultrasound images of the ovarian tumor. The set of ultrasound images may for example be obtained by an ultrasound examination of an individual. Thus, a set of images may comprise a plurality of images that are obtained during one ultrasound examination of an individual.

The method may 27urtherr comprise a step of providing the set of ultrasound images to a trained multi-class neural network. The neural network may have been trained according to the method for training a multi-class neural network for aiding in diagnosing ovarian tumors using ultrasound images, as disclosed elsewhere herein.

In this way, a malignancy score of the ovarian tumor may be determined.

In one embodiment of the present disclosure, the step of providing comprises obtaining probability estimates, for each diagnostic classification of a hierarchical ontology, from the trained multi-class neural network.

In one embodiment of the present disclosure, the method comprises a step of calibrating the obtained probability estimates.

In one embodiment of the present disclosure, the method comprises a step of computing the risk of malignancy for each ultrasound image of the set, by summing the probability estimates attributed to malignant sub-diagnosis groups.

In one embodiment of the present disclosure, the method comprises a step of calculating the final malignancy score of the set from the malignancy score of each image of said set.

In one embodiment of the present disclosure, the method comprises a step of applying a decision rule to predict a case as malignant or benign based on whether the final malignancy score of the set exceeds a predetermined operating point.

In one embodiment of the present disclosure, the set of ultrasound images have been obtained by an ultrasound examination of the ovarian tumor.

In one embodiment of the present disclosure, the method is a computer-implemented method.

In a further aspect, the present disclosure relates to a computer program product comprising instructions which, when the program is executed by a computer, cause the computer to carry out the method of determining a malignancy score of an ovarian tumor as disclosed elsewhere herein, or the method for training a multi-class neural network for aiding in diagnosing ovarian tumors using ultrasound images, as disclosed elsewhere herein.

In a further aspect, the present disclosure relates to a computer-aided detection (CAD) system for diagnosing an ovarian tumor of a patient using ultrasound image data of said patient.

In one embodiment of the present disclosure, the system comprises a data reception module arranged to receive the ultrasound image data. The data reception module may for example be arranged to exchange data with an ultrasound system and/or be an ultrasound system (i.e. a system capable of performing ultrasound examinations).

In one embodiment of the present disclosure, the system comprises a lesion diagnosis module comprising a computer-readable medium having stored thereon a multi-class neural network for diagnosing ovarian tumors using ultrasound images. The multi-class neural network may have been trained according to the method for training a multi-class neural network for aiding in diagnosing ovarian tumors using ultrasound images, as disclosed elsewhere herein.

In one embodiment of the present disclosure, the system comprises a malignancy scoring model, configured to calculate a malignancy score of the ultrasound image data.

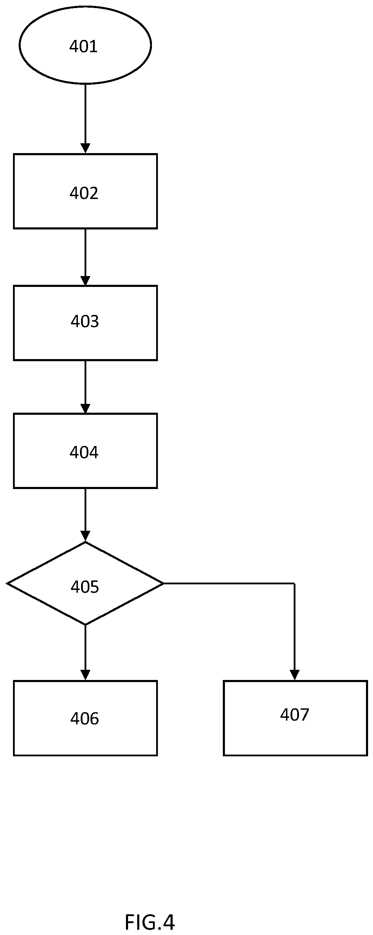

A process of calculating a malignancy scores by the Computer-Aided Detection (CAD) system, according to an embodiment of the present disclosure, is shown in FIG. 4. This process utilizes a trained multi-class neural network to provide a quantifiable measure of malignancy risk for each case.

The steps involved, as shown in the example of Fig. 4, are: obtain probability estimates, calibrate probability

13

estimates, compute risk of malignancy for each image, determine the malignancy score for the case, and apply the decision rule for diagnosis. However, other examples comprise only one or more of said steps, or even other steps. In one example, the system is arranged to receive ultrasound image data, compute a risk, and calculate a malignancy score of the ultrasound image data.

In one embodiment of the present disclosure, the neural network may be arranged to process each image of a case (i.e. each image data of a set of ultrasound images) and outputs probability estimates for each diagnostic class, such as according to a hierarchical ontology.

These probabilities may be obtained from the network's final layer, which may have been trained to classify the ultrasound images into various classes, including benign and malignant subtypes.

The network may be arranged to use the exponential moving average (EMA) of its weights, which helps in stabilizing and improving the prediction accuracy. To enhance the reliability of these probability estimates, they may be calibrated using temperature scaling or Platt scaling. This step may be arranged such that it adjusts the raw output probabilities to better match the empirical probabilities, thus improving the model's confidence estimates.

Mathematically, if p is the raw probability and T is the temperature scaling parameter, then the calibrated probability p_cal is given by:

$$p\_cal = softmax(p / T)$$
for temperature scaling, or by:

$$p\_cal = 1 / (1 + exp(A * p + B))$$
for Platt scaling, where A and B are parameters learned from a validation set. The risk of malignancy for each image is computed by summing the calibrated probabilities attributed to all malignant sub-diagnosis groups.

If P_malignant represents the set of probabilities corresponding to malignant diagnoses, then the risk R_image for an image is calculated as: R_image = sum(P_malignant)

The malignancy score for an entire case can be determined by averaging the malignancy risks computed for each image in that case. If N_images is the number of images in a case and Ri is the risk for the i-th image, then the malignancy score M_case for the case is: M_case = (1 / N_images) * sum(Ri) for i = 1 to N_images

Finally, a decision rule can be applied to predict whether a case is malignant or benign. This involves comparing the malignancy score M_case for a case against a predetermined threshold or operating point, for example 0.5. If M_case exceeds this threshold, the case is predicted as malignant; otherwise, it is predicted as benign. The malignancy score calculation process, as outlined above and depicted in Fig. 4, provides a systematic and quantifiable approach to assessing the likelihood of malignancy in ovarian tumors using ultrasound imaging. This method enables the system to deliver accurate and reliable diagnostic predictions, crucial for effective clinical decision-making.

In one embodiment of the present disclosure, the multi-class neural network has been trained according to the method for training a multi-class neural network for aiding in diagnosing ovarian tumors using ultrasound images as disclosed elsewhere herein.

In one embodiment of the present disclosure, the system is arranged to carry out the method of determining a malignancy score of an ovarian tumor as disclosed elsewhere herein. The malignancy score may for example be computed from the sum of logits of the multi-class neural network.

In one embodiment of the present disclosure, the system comprises an integration interface arranged to facilitate data exchange and result sharing with electronic health records (EHR) systems and/or diagnostic workstations;

In one embodiment of the present disclosure, the system comprises a user interface configured for providing healthcare professionals with interactive tools for review of a case and decision support, such as wherein the user interface is adaptable to clinical workflows.

In one embodiment of the present disclosure, the system comprises a ROI detector module arranged to annotate regions-of-interest (ROI) in the images.

In one embodiment of the present disclosure, the system comprises an image preprocessing unit configured to preprocess the ultrasound image data, such as wherein the image preprocessing unit is configured to process said image data by remove image artifacts and/or perform standardization of images, such as image size and/or quality.

In one embodiment of the present disclosure, the system comprises an ultrasound device, arranged to obtain ultrasound images of a patient, and to provide the obtained ultrasound images to the data reception module.

**Description of the drawings**

In the following, embodiments and examples will be described in greater detail with reference to the accompanying drawings:

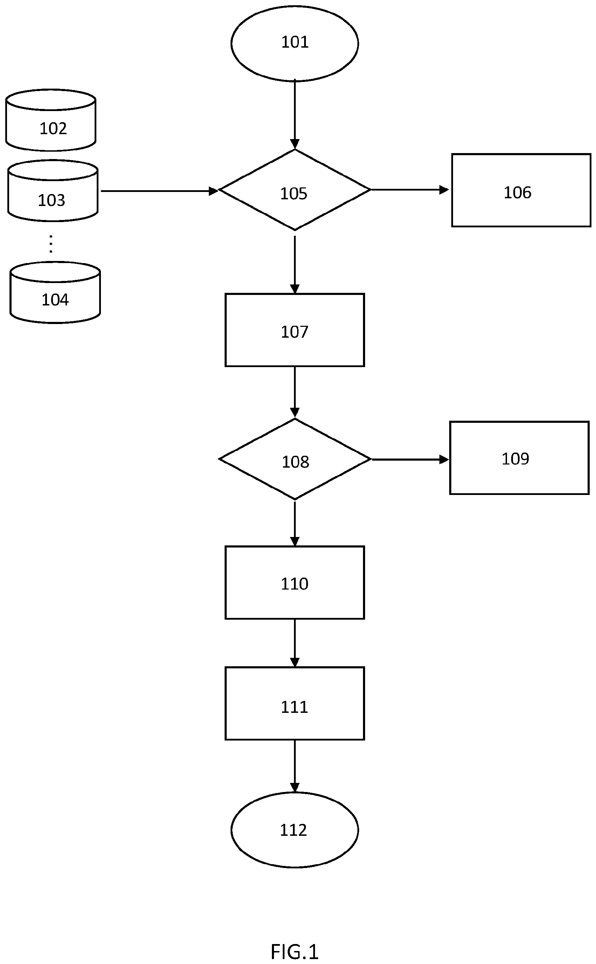

Fig. 1 is a protocol sequence diagram of the data curation process, according to an embodiment of the present disclosure. The data curation process for the Computer Aided Detection (CAD) begins with collection (101) of ultrasound images from the different centers (102, 103, 104). The collected data should preferably encompass images captured from a wide range of ultrasound device types and. The data can adhere to certain criteria (105), such as temporal distribution, exam composition and ultrasound device diversity. Data that do not fit the criteria may be excluded (106). After data is collected (107) and filtered (105,106) each image undergoes a quality assessment (108). If the image does not pass this quality assessment it is excluded (109). Images that pass the quality assessment proceed to the next step of annotation region of interest (ROI)(110). Subsequently each image undergoes expert annotation / diagnosis (111) which is the end of the data curation (112).

Fig. 2 is a diagram of the hierarchical diagnostic ontology, according to an embodiment of the present disclosure.

At the highest level, the ontology bifurcates diagnoses into the fundamental categories of benign (201) and malignant (202), forming the basis of the diagnostic process. The second level expands this into ten distinct categories of ovarian conditions, further classified into benign; Endometrioma (203), Dermoid (204), Other common benign (205) Solid benign (206), Cystadeno(fibro)ma (207) and Rare benign (208) and malignant subtypes Borderline (serious) (209), Borderline (mucinous intestinal) (210), Ovarian cancer (epithelial) (211), Ovarian cancer (non-epithelial) (212), Metastasis (213). The third level details eighteen categories, offering a more intricate classification based on histological and morphological traits Endometrioma (214), Dermoid (215), Simple / inclusion cyst (216), Paraovarian cyst (217), Functional cyst (218), Peritoneal cyst (+) (219), Solid benign (220), Cystadenoma (serious) (221), Cystadenoma (mucinous) (222), Cystadenofibroma (223), Rare benign (224), Borderline (serious) (225), Borderline (mucinous intestinal) (226), Ovarian cancer (serious) (227), Ovarian cancer (mucinous) (228), Ovarian cancer (endometroid / clear-cell) (229), Ovarian cancer (non-epithelial) (230), Metastasis (colorectal /pancreas) (231), Metastasis (other) (232).

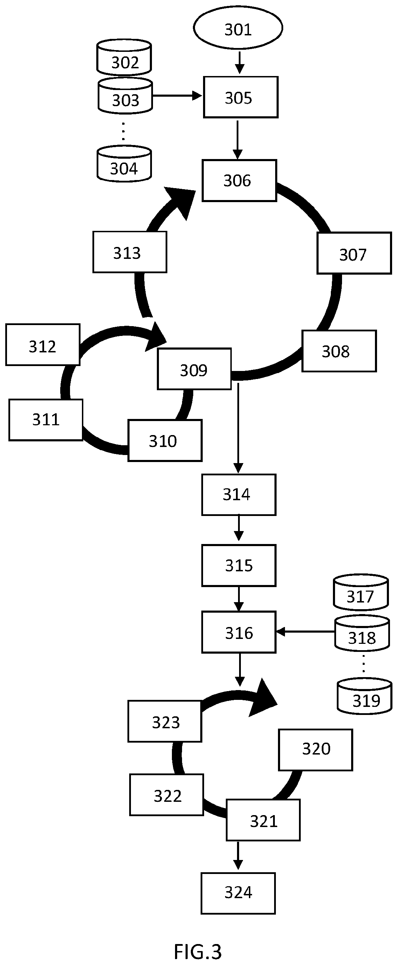

Fig. 3 illustrates the learning (or training) phase of the neural network, according to an embodiment of the present disclosure.

The process starts (301) with data being collected and preprocessed from N different diagnostic centers (302, 303, 304). The preprocessing of ultrasound image data for the CAD system can involve steps to standardize, enhance, and secure the images before they are inputted into the neural network for training (305). The next step is the training process. It is an implementation of a leave-one-out cross-validation loop (306). In this loop, data from one center is left out for validation purposes, while the remaining data is used for training (e.g. training data). The training data may then be split according to a predetermined ratio, such as 90%/10% where 10% is used for validation and hyperparameter selection. This approach is repeated N times, each time leaving out data from a different center. Each fold of the cross-validation typically has its own validation set (e.g. 10%), and also the held out center (the test set). Hyperparameters for training the neural network are chosen at the start of each training iteration (307). The learning rate, the learning schedule, the batch size , the architecture of the network and the training length are searched. The hyperparameters are chosen according to a grid search or Bayesian Optimization.

Network weights are initialized either randomly or using pre-trained weights (308). The neural network is trained through several iterations. Key steps in each iteration are hierarchical data sampling (309), forward pass (310), loss calculation (311) and back propagation (312). After training with each subset of data multiple times to search for good hyperparameters, a statistical analysis (314) of the hyperparameters is conducted to identify the set of hyperparameters (315) that provide the highest average performance (measured in e.g. AUC) and the least variance across all validation sets. Thus, the optimal set of hyperparameters may be the combination resulting in the highest AUC and the lowest variance.

Once the most robust hyperparameters are identified, the entire dataset (317, 318, 319) is preprocessed (316) and used for the final model (324) training. This training also employs hierarchical data sampling (320), forward pass (321) multi-class focal loss (322) and backpropagation (323).

Fig. 4 illustrates the process for calculating the malignancy score for a case, according to an embodiment of the present disclosure,Calculation of the malignancy scores in the Computer-Aided Detection (CAD) system utilizes the trained multi-class neural network to provide a quantifiable measure of malignancy risk for each case. The calculations start (401) by obtaining probability estimates (402), calibrate probability estimates, compute the risk of malignancy for each image (403), determine the malignancy score (404) for the case, and apply the decision rule (405) for diagnosis. If the score is ≥ 0.5 the case is malignant (406) and if the score is < 0.5 the case is benign (407).

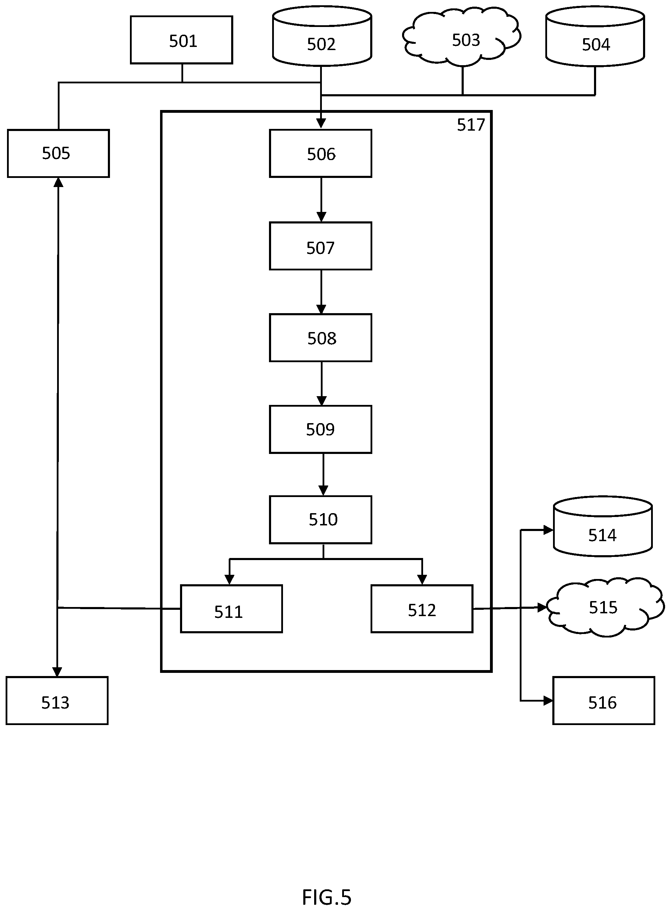

Fig. 5 is an example of a computer aided detection (CAD) system, the system may implement various services, systems, and/or methods as disclosed herein.

The ultrasound Computer-Aided Detection (CAD) (517) system integrates a series of specialized modules and a neural network to provide an effective tool for diagnosing ovarian tumors using ultrasound imaging. Each component plays a role in processing and analyzing the image data, culminating in a reliable malignancy. A data reception module (506) that receives the ultrasound images from various sources such as image generating devices (501), PACS system (502), Cloud service (503) and other sources (504); an image processing unit (507) which applies several techniques, including artifact removal algorithms and normalization methods, to standardize image quality across different ultrasound device outputs. This step maintains consistency in the data fed into the neural network; An ROI Detector Module (508) programmed to annotate regions-of-interest (ROI) within the images; An AI lesion diagnosis module (509) which utilizes the neural network trained as outlined elsewhere herein; A malignancy scoring algorithm (510) that calculates a malignancy score for each case. It averages the malignancy scores derived from each image in a case and uses a threshold determination mechanism to make the final diagnosis. The user interface (511) of the CAD system provides interactive tools designed for healthcare professionals, offering features such as case review, decision support and navigation through diagnostic results. The system integration interface (512) ensures seamless data exchange and result sharing with electronic health records (EHR) systems and diagnostic workstations. The system has a display device (505) and a Keyboard /input device (513). Generated data may be stored on a PACS system (514), a cloud service (515) and or EHS (516).

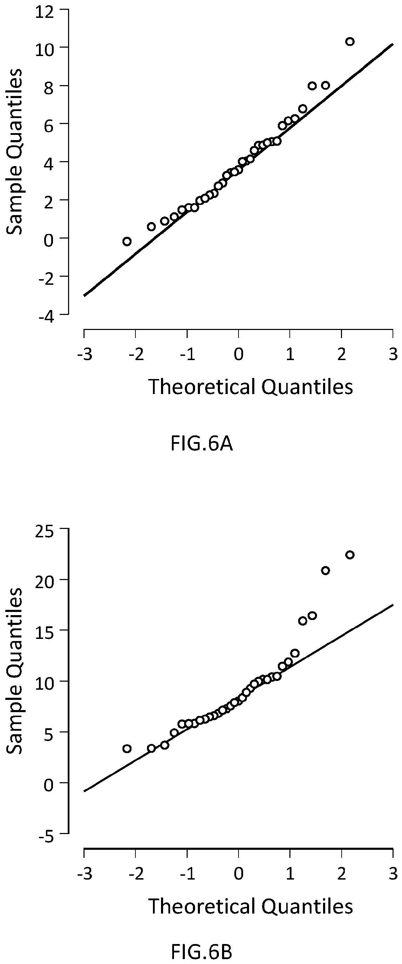

Fig. 6 shows Q-Q plots of the differences in paired F1 scores between the models and expert examiners (Fig. 6A) and the models and non-expert examiners (Fig. 6B). The plots support the results of the Shapiro-Wilk test, indicating that the differences in paired F1 scores between the models and expert examiners are approximately normally distributed, but not for the models and non-expert examiners.

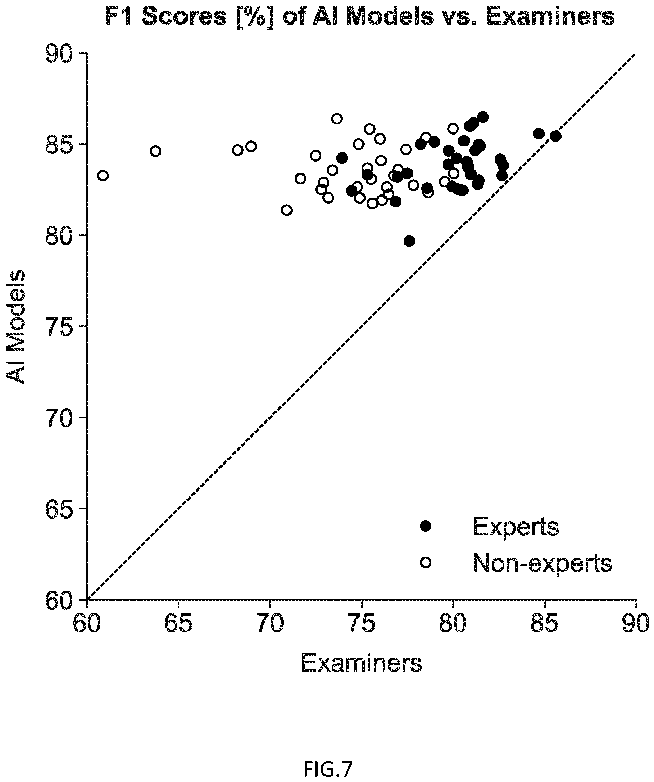

Fig. 7 shows a comparison of individual human examiners and a multi-class neural network, according to an embodiment of the present disclosure. Each dot represents the set of cases assessed by an individual human examiner. The x-axis signifies the F1 score that a given examiner achieved on a set of cases assigned to them. The y-axis signifies the F1 scores of the AI models on the same set of cases. Dots above the dashed-black line indicate that the AI models performed better than the individual examiner on the matching set of cases.

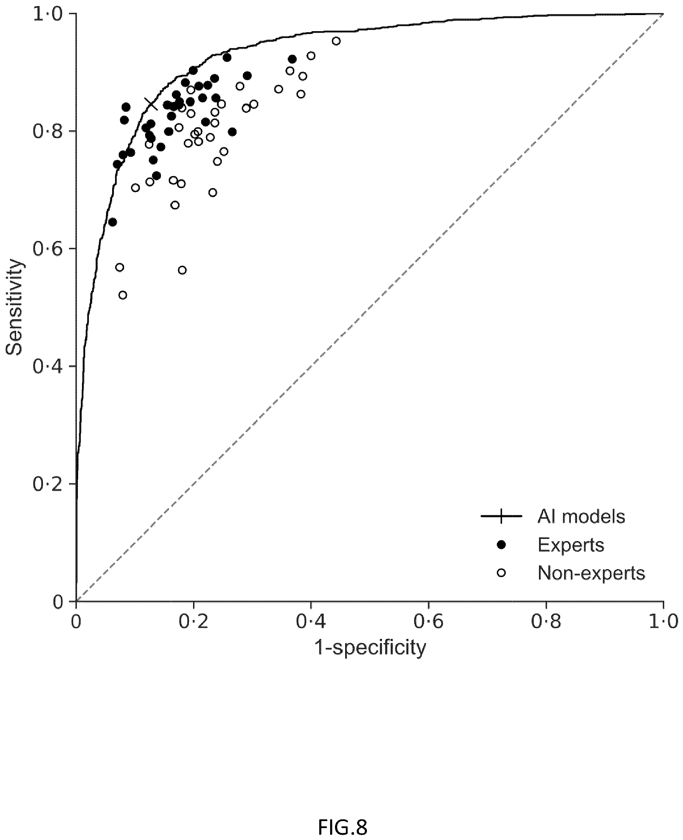

Fig. 8 shows a model ROC curve and human examiner performance. The model performance is given as a receiver operating characteristics (ROC) curve. Each dot represents a human examiner, with non-experts in white and experts in black. The models were evaluated on 2718 cases, while each human examiner assessed only a subset of cases. Hence, while multiple individual expert examiners seem to outperform, or perform on pair with, the models, by lying above the ROC curve of the models, only a single expert examiner outperforms the models on the same set of cases.

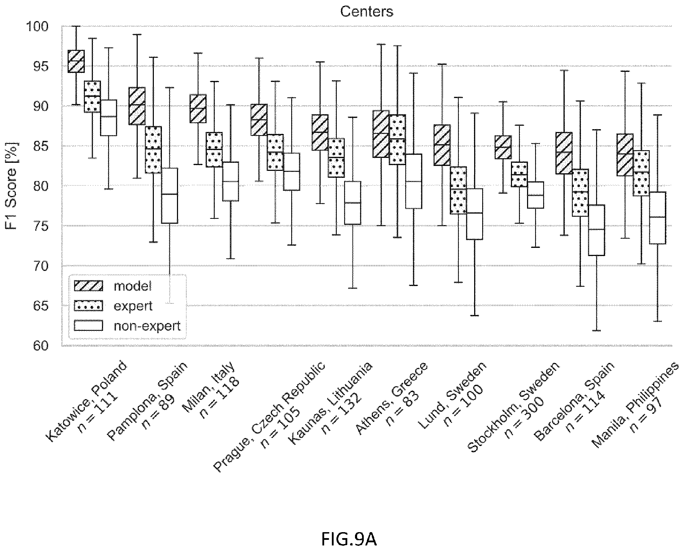

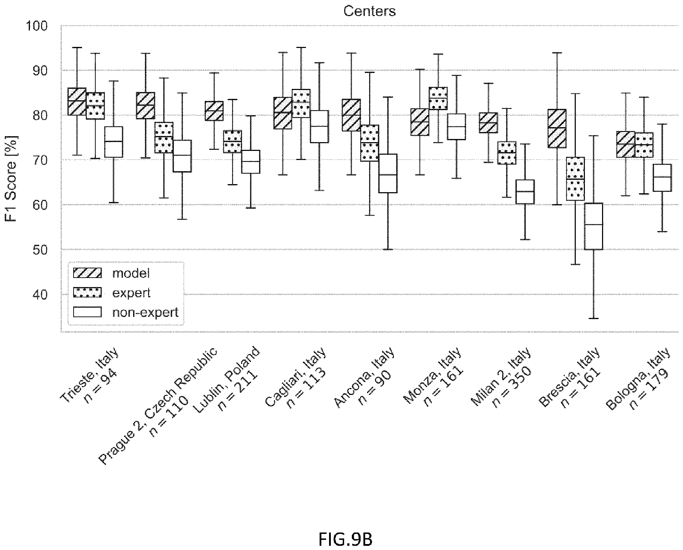

Fig. 9 shows a comparison of AI models, trained according to an embodiment of the present disclosure, and expert and non-expert examiners, when slicing the data according to medical center.

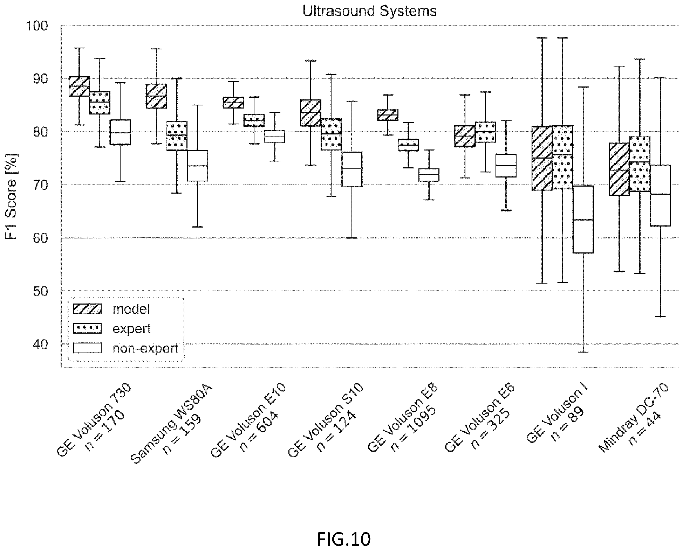

Fig. 10 shows a comparison of AI models, trained according to an embodiment of the present disclosure, and expert and non-expert examiners, when slicing the data according to ultrasound system, limited to the eight most common systems.

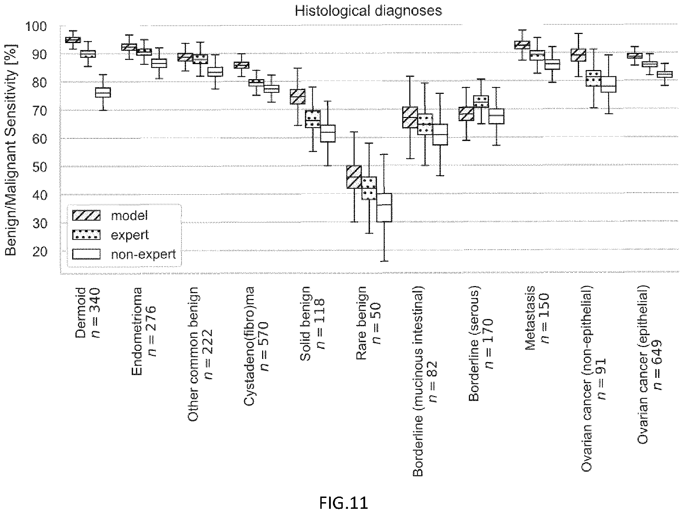

Fig. 11 shows a comparison of AI models, trained according to an embodiment of the present disclosure, and expert and non-expert examiners, when slicing the data according to histological diagnosis.

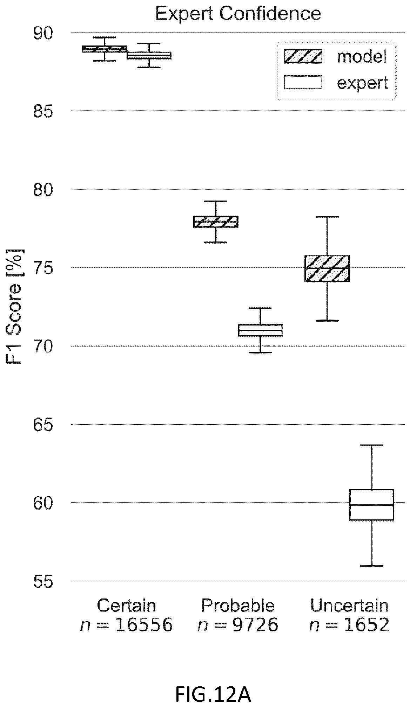

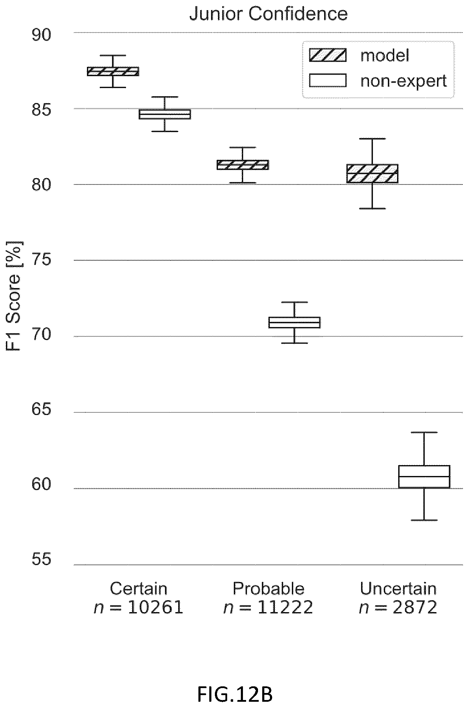

Fig 12 shows F1 scores for expert examiners vs a model, trained according to an embodiment of the present disclosure, (Fig. 12A) and non-expert examiners vs model, trained according to an embodiment of the present disclosure, (Fig. 12B), partitioned by the examiner's confidence in their assessment. For each level of confidence (certain, probable, uncertain), all assessments made by experts (left) and non-experts (right) were pooled with the corresponding level of confidence.

**Examples**

Example 1 training of multi-class neural networks

The ability of a transformer-based DNN model to distinguish between benign and malignant ovarian tumors in ultrasound images and the model's ability to generalize across different patient populations, centers, and ultrasound systems was evaluated in a study comprising 17119 ultrasound images retrospectively collected from 3652 women with an ovarian lesion (2224 benign, 1428 malignant) from 20 centers in eight countries. A total of 2718 cases were externally reviewed by a minimum of seven experts and six non-expert examiners. For each center, the transformer-based DNN model was trained using data from the remaining 19 centers and compared the models' performance with that of expert and nonexpert examiners in terms of accuracy, sensitivity, specificity, and F1 score.

Data acquisition and pre-processing

Transvaginal and transabdominal ultrasound images from women with an ovarian lesion, examined between 2006 and 2021 at 20 secondary or tertiary referral centers for gynaecological ultrasound in eight countries were included in the assessment.

The images were acquired by examiners with varying levels of training and experience, using 21 different commercial ultrasound systems from nine manufacturers, primarily GE (91.8%), followed by Samsung (4.8%), Philips (1.4%), and Mindray (1.2%). Participating centers were requested to provide images of at least 50 consecutive malignant cases and at least 50 benign cases examined just prior to or after each malignant case, to ensure a similar temporal distribution and avoid bias from potential variations in diagnostic practices or equipment over time. This enrichment strategy was designed to ensure an adequate representation of malignant cases, thereby more effectively capturing rare pathologies while minimizing potential biases. The images were submitted in JPEG format. After excluding 4.9% (n = 188/3840) of the cases (96 benign, 92 malignant) due to unsatisfactory image quality (e.g., lesions that could not be identified, lesions with blurred margins, and lesions that were only partially visible), 17119 ultrasound images (10626 grayscale, 6493 Doppler) representing 3652 cases remained for analysis. Out of these 3652 cases, 3419 were patients who had undergone surgery, including histological assessment, within 120 days of their ultrasound examination. The remaining 233 patients had been managed conservatively with ultrasound follow-up until the resolution of the lesion, or for at least three years without a malignant diagnosis, and were thus regarded as benign. The median number of images per case was four.

Four gynaecology residents were tasked with manually selecting a rectangular region of interest (ROI) in each image, whereby the lesion was centrally located and occupied most of the image. The involvement of gynaecology residents aimed to avoid potential bias and dependence on advanced domain expertise not always present in routine clinical practice. The images were cropped according to the annotated ROIs for both model training and evaluation. The residents also marked artifacts, e.g., text, inside the ROI for removal.

Human examiner review

The assessments of 66 human examiners, comprising 33 ultrasound experts and 33 non-experts were collected. An 'expert' examiner was defined as a physician who works with second or third opinion gynaecological ultrasound imaging, and who has at least five years' experience or annually assesses at least 200 patients with a persistent ovarian lesion. Among the experts, the median experience in gynaecological ultrasound imaging was 17 years, with a median of 10 years as second or third opinion. Most experts (91%, n = 30/33) were affiliated with a gynaecologic oncology referral center, 61% (n = 20/33) performed over 1500 gynaecological ultrasound scans annually, and 64% (n = 21/33) reported seeing more than 200 patients with a persistent ovarian lesion each year. 'Non-expert' examiners, all of whom practiced gynaecological ultrasound imaging, had a median experience of 5 years, and 52% (n = 17/33) were affiliated with a gynaecologic oncology referral center. Furthermore, 24% (n = 8/33) of non-experts served as second or third opinion. When presented with a case, the examiner was asked to classify the lesion as benign or malignant, and rate their confidence in the assessment, as certain, probable, or uncertain. To prevent bias from previously seen cases, none of the examiners were asked to review cases originating from their own centers. A total of 2718 cases (1576 benign, 1142 malignant) were assessed by at least 7 expert and 6 non-expert examiners, for a total of 52289 assessments. The median number of cases assessed by each expert and non-expert examiner was 708 and 630 respectively.

Model training

The dataset was employed to train a series of 19 transformer-based DNN models using DeiT architecture initialized with ImageNet pretraining. Applying a leave-one-out cross-validation scheme, for each center in turn, that center was isolated as the test set and the model was given the cases from the remaining centers for training. The cases from the remaining centers were randomly split into a training (90%) and a validation (10%) set, with the validation set used for selection of the hyperparameters. The goal was to differentiate between benign and malignant lesions, the models were trained to discern ten different histological categories within the benign and malignant classes. This was done in order to leverage the richer information contained in the specific histological diagnoses to improve performance. The models were trained using multi-class focal loss which, compared to the standard cross-entropy loss, encourages the model to assign greater importance to often misclassified examples. After training, the multi-class DNN models provided probability estimates for each of the ten diagnostic classes. The risk of malignancy was computed for an image by summing up the probabilities for the five malignant classes. The malignancy score for a case was then computed as the average of the malignancy scores of its images. A case was considered malignant if its malignancy score exceeded a given cut-off point. Unless otherwise stated, we used the default cut-off point of 0.5.

Evaluation procedure

A rigorous assessment of the diagnostic performance of the models was conducted via separate test sets, each containing only data from the center withheld during training, to avoid overly optimistic results commonly seen in medical machine learning.

The models were compared to expert and non-expert examiners using the F1 score as the primary metric because both precision and recall are considered. However, as disclosed herein, other performance metrics may be used. The F1 score is the harmonic mean of the precision (i.e., positive predictive value) and the recall (i.e., sensitivity):

$$F1 = 2\frac{PPV * sensitivity}{PPV + sensitivity}$$

Metrics were calculated at the case level, as opposed to image-wise. In addition to the F1 score, accuracy, sensitivity, and specificity scores were also reported. The primary evaluation compared the performance of AI models with each individual examiner's assessments on matched case sets. When calculating the diagnostic performance of the models, the originating center was identified for each case and used the model that had not been exposed to cases from that center during training.

Results

The AI models were evaluated by comparing their diagnostic performance against expert and non-expert examiners. The diagnostic performance, expressed as accuracy, sensitivity, specificity, and F1 score, is shown in Table 1.

| Table 1 | Human resources | Accuracy | Sensitivity | Specificity | F1 |
|---|---|---|---|---|---|
| Single non-expert | 1 | 77.67 (76.34-78.99) | 78.50 (76.46-80.48) | 77.08 (75.30-78.83) | 74.70 (73.03-76.30) |
| Single expert | 1 | 82.56 (81.35-83.74) | 82.30 (80.44-84.12) | 82.74 (81.15-84.30) | 79.85 (78.32-81.33) |
| Current practice | 1.52 (1.51-1.54) | 81.98 (80.76-83.19) | 73.78 (71.62-75.86) | 87.92 (86.54-89.24) | 77.47 (75.81-79.08) |
| AI model | 0 | 86.13 (85.03-87.20) | 84.59 (82.78-86.35) | 87.24 (85.83-88.62) | 83.66 (82.25-85.04) |

The models consistently outperformed both expert and non-expert examiners across all metrics, p < 0.0001 (Table 1).

Paired comparisons of the F1 scores between each human examiner and the AI models showed that the models outperformed all non-expert examiners and all but one expert examiner (Fig. 7).

The ROC curve (Fig. 8) illustrates that the AI models outperformed both mean expert and non-expert performance over a range of cut-off points.

To directly compare the sensitivity and specificity of our AI models with the performance of human expert and non-expert examiners, the results of the AI models at matching cut-off points are presented in Table 2. The AI models exhibit superior sensitivity (89.24% vs. 82.30%; $\Delta$ = 6.94 [95% CI, 5.05-8.87, p < 0.0001]) when specificity is held constant at the expert level (82.74%). They also excel in specificity (88.83% vs. 82.74%; $\Delta$ = 6.08 [95% CI, 4.60-7.57, p < 0.0001]) when sensitivity is set at the expert level (82.30%). When compared to non-expert examiners, the disparities are even more substantial, with differences of 14.07 and 13.47 percentage points in sensitivity and specificity, respectively.

| Table 2 | Experts | Model | $\Delta$ | p-value |
|---|---|---|---|---|
| Sensitivity at expert specificity (82.74) | 82.30 (80.44-84.12) | 89.24 (87.69-90.74) | 6.94 (5.05-8.87) | < 0.0001 |
| Specificity at expert sensitivity (82.30) | 82.74 (81.15-84.30) | 88.83 (87.49-90.13) | 6.08 (4.60-7.57) | < 0.0001 |
| | Non-experts | | | |
| Sensitivity at non-expert specificity (77.08) | 78.50 (76.46-80.48) | 92.56 (91.28-93.83) | 14·07 (12.10-16.06) | < 0.0001 |
| Specificity at non-expert sensitivity (78.50) | 77.08 (75.30-78.83) | 90.55 (89.30-91.74) | 13·47 (11.76-15.20) | < 0.0001 |

The AI models consistently outperformed both expert and non-expert examiners, except for two centers, Monza and Cagliari (Fig. 9). The model performance across different ultrasound systems were also examined and it was found that the AI models exhibited robust performance, matching or surpassing the performance of expert examiners, irrespective of the ultrasound manufacturer or system used (Fig. 10). Model performance across histological diagnoses was assessed, as illustrated in Fig. 11, where the AI models exhibit superior performance compared to human expert and non-expert examiners, even in cases known to be challenging to classify, such as cystadeno(fibro)mas, solid lesions, and mucinous intestinal borderline tumors.

A strong correlation between examiner confidence and performance was noted, with a sharp decrease in performance for uncertain assessments. In contrast, the AI models demonstrated only a modest decline in performance in these challenging cases (Fig. 12).

It is understood that those skilled in the art may contemplate various modifications and alternative embodiments of the invention without deviating from the spirit and scope delineated in the appended claims. The present invention is not confined to the disclosed embodiments, and alternative configurations are intended to be included within the expansive scope of the claims.

The described embodiments are provided for illustrative purposes, and the inventive concept can be implemented in numerous ways. Variations in the arrangement and configuration of the disclosed elements or the incorporation of additional elements may be employed to achieve the same or similar results.

References to "one" or "a" particular element, component, or feature throughout the specification are not intended to be limiting, and such references encompass "one or more" instances unless explicitly stated otherwise. References to a "plurality" of elements, components, or features are intended to encompass any suitable number, including but not limited to two or more.

The terms "or" and "and/or" are to be interpreted inclusively, encompassing any single element or any combination thereof. Thus, expressions such as "A, B, or C" or "A, B, and/or C" signify "any of the following: A; B; C; A and B; A and C; B and C; A, B, and C." This inclusive interpretation applies unless a combination of elements, functions, steps, or actions is inherently mutually exclusive.

The terms used in the claims, such as "comprising," "including," or "having," should be construed broadly, allowing for the presence of one or more additional elements, steps, or features not specifically disclosed. Generic references, such as "anything," "anyone," or "any," are inclusive and not limiting, permitting the application of the invention to any suitable context.

The use of reference numerals in the specification and drawings is for clarity and convenience, not limiting the scope of the invention. The same reference numerals may be used to designate similar or identical elements, their use not constraining the invention.

The language used throughout this specification, including the claims, is non-limiting and for descriptive purposes. Variations and equivalents of the disclosed features are acknowledged and contemplated within the broad scope of the invention.

The specification and claims herein enable a person skilled in the art to make and use the invention without undue experimentation. The terms and phrases are to be interpreted in an open-ended fashion, allowing for their broadest possible construction.

It is recognized that future technological developments may necessitate modifications, and this patent application is intended to cover such developments falling within the scope of the claims. All references cited throughout this specification are incorporated by reference in their entirety.

This disclosure is not an exhaustive review of prior art, and the invention is not limited to specific prior art references. The specification and claims are written to enable and describe the invention for a person skilled in the art. The inclusion of specific features in the claims is not intended to imply essentiality to the invention.

**Items**

1. A method for training a multi-class neural network for aiding in diagnosing ovarian tumors using ultrasound images, the method comprising:

a. obtaining ultrasound image data from a plurality of diagnostic centers, each center providing images of both malignant and benign ovarian tumors, the image data comprising a plurality of sets, wherein each set comprises image data of an ultrasound examination of an individual patient;
b. annotating each image of the image data, comprising labeling the image data with histological diagnoses organized into a hierarchical ontology;
c. training a series of initial neural networks to perform multi-class classification wherein said training comprises a leave-one-out cross validation, wherein, for each round of said cross validation, a different center is used as hold-out test data, and a new initial neural network is trained using training data that is derived from the remaining image data;
d. evaluating the performance of each initial neural network of the series of initial neural networks;
e. identifying an optimal set of hyperparameters that yield the most consistent performance across all hold-out centers, based on the performance and the hyperparameters of each initial neural network; and

f. training a final neural network using the optimal set of hyperparameters.

2. The method according to item 1, wherein each set comprises a plurality of images, such as grayscale and/or doppler images.

3. The method according to any one of the preceding items, wherein each set comprises a mixture of grayscale and doppler images.

4. The method according to any one of the preceding items, wherein the centers are medical facilities located in different cities and/or countries.

5. The method according to any one of the preceding items, wherein the image data from each center has been generated by a different combination of ultrasound examiner, ultrasound system, imaging modality and the presence of calipers.

6. The method according to any one of the preceding items, wherein the image data is provided in a DICOM or a JPEG format.

7. The method according to any one of the preceding items, wherein the image data comprises, from each center, both malignant and benign cases.

8. The method according to any one of the preceding items, wherein the image data of each center has a ratio of malignant and benign cases in the range between 30%-70%:30%-70%, such as 45%-55%:45%-55%.

9. The method according to any one of the preceding items, wherein the image data comprises a plurality of ultrasound examiners, ultrasound systems, imaging modalities and/or information whether there is a presence of calipers in the image or not.

10. The method according to any one of the preceding items, wherein the step of annotating comprises labeling each image of the image data with histological diagnoses that correspond to a pre-defined hierarchical ontology.

11. The method according to item 10, wherein the hierarchical ontology comprises multiple hierarchical levels.

12. The method according to any one of the preceding items, wherein the step of annotating comprises annotating each image of the image data with a region-of-interest (ROI).

13. The method according to any one of the preceding claims, wherein the step of annotating comprises the use of an artificial intelligence (AI) detector, such as for annotating each image of the image data with a region-of-interest (ROI).

14. The method according to any one of the preceding items, wherein the method further comprises a step of preprocessing the image data, before the step of training, such as after the step of annotating.

15. The method according to item 14, wherein the preprocessing comprises anonymization, cropping, resizing, and/or normalization of pixel values.

16. The method according to any one of the preceding items, wherein the hierarchical ontology comprises a plurality of levels of granularity.

17. The method according to any one of the preceding items, wherein the hierarchical ontology comprises at least three levels of granularity, such as at least four level of granularity.

18. The method according to any one of the preceding items, wherein the hierarchical ontology comprises a fourth level of ovarian tumor subtypes and/or histological and morphological traits, a third level of ovarian lesion classification, a second level of detailed classification, and a first level of binary classification.

19. The method according to item 18, wherein the third level comprises at least 10 classifications, such as 18 classifications, and wherein the second level comprises at least 5 classifications, such as 10 classifications, and wherein the first level comprises 2 classifications.

20. The method according to any one of the preceding items, wherein the step of evaluating comprises calculating, for each initial neural network, a performance metrics, such as the area under the received operating characteristics (AUC), such as for binary classification of each set.

21. The method according to any one of the preceding items, wherein the step of evaluating comprises using each hold-out test data to evaluate the performance of the respective initial neural network

22. The method according to any one of the preceding items, wherein the step of identifying comprises identifying the optimal set of hyperparameters that yields the highest average performance and least variance across all hold-out centers.

23. The method according to any one of the preceding items, wherein the step of identifying comprises identifying the optimal set of hyperparameters by performing a statistical analysis of the performance and the hyperparameters of each initial neural network.

24. The method according to any one of the preceding items, wherein the step of identifying comprises identifying the optimal set of hyperparameters, wherein the hyperparameters comprise the group including the learning rate, the learning schedule, the batch size, the architecture of the network and/or the training length.

25. The method according to any one of the preceding items, wherein the initial and/or final neural network is a convolutional neural network or a vision transformer, such as a data efficient image transformer.

26. The method according to any one of the preceding items, wherein the network weights of the initial neural networks and/or the final neural network are initialized either randomly, or by transfer learning, such as using pre-trained weights from models trained on natural scenes, such as ImageNet, from other medical imaging modalities e.g. x-ray, other medical domains, e.g. mammograms, or other ultrasound.

27. The method according to any one of the preceding items, wherein each initial neural network and/or the final neural network is trained using multi-class focal loss.

28. The method according to item 27, wherein the multi-class focal loss is calculated for each image, such as in the training data and/or the batch sampled from the training data.

29. The method according to any one of the preceding items, wherein the training data is derived from the image data, for each round of the cross-validation.

30. The method according to any one of the preceding items, wherein the image data is, for each round of the cross-validation, randomly split, according to a predetermined ratio, into a training set used for deriving the training data and a validation set used for selection of a learning rate.

31. The method according to any one of the preceding items, wherein the final neural network is trained using the image data, such as the full image data.

32. The method according to any one of the preceding items, wherein the final neural network is arranged for providing a decision support for diagnosing ovarian cancer.

33. The method according to any one of the preceding items, wherein the training data is derived from the image data by hierarchical sampling.

34. The method according to any one of the preceding items, wherein the method is a computer-implemented method.

35. The method according to item 33, wherein the hierarchical sampling is arranged such that the training data comprises a balanced distribution of the categories of the image data.

36. The method according to item 35, wherein the balanced distribution of data is arranged such that the training data comprises each category in terms of ovarian lesion classification, imaging modality, device type, and the presence of calipers.

37. The method according to any one of items 35-36, wherein the hierarchical sampling comprises use of a sampling weight for each category, wherein said sampling weight is determined by the inverse of the frequency of each category.

38. The method according to any one of items 35-37, wherein the sampling weights are determined by the inverse of the frequency of each category multiplied by a factor E, thereby enriching under-represented categories of the image data.

39. Use of a trained multi-class neural network for diagnosing ovarian tumors using ultrasound images.

40. The use according to item 39, wherein the multi-class neural network has been trained according to any one of items 1-38.

41. A method of determining a malignancy score of an ovarian tumor, the method comprising:

a) obtaining a set of ultrasound images of the ovarian tumor;
b) providing the set of ultrasound images to a trained multi-class neural network that has been trained according to any one of items 1-38, thereby determining the malignancy score of the ovarian tumor.

42. The method according to item 41, wherein the step of providing comprises:
obtaining probability estimates, for each diagnostic classification of a hierarchical ontology, from the trained multi-class neural network.

43. The method according to item 42, wherein the method further comprises a step of calibrating the obtained probability estimates.

44. The method according to any one of items 42-43, wherein the method further comprises a step of computing the risk of malignancy for each ultrasound image of the set, by summing the probability estimates attributed to malignant sub-diagnosis groups.

45. The method according to item 44, wherein the method further comprises a step of calculating the final malignancy score of the set from the malignancy score of each image of said set.

46. The method according to item 46, wherein the method further comprises a step of applying a decision rule to predict a case as malignant or benign based on whether the final malignancy score of the set exceeds a predetermined operating point.

47. The method according to any one of items 41-46, wherein the set of ultrasound images have been obtained by an ultrasound examination of the ovarian tumor.

48. The method according to any one of items 41-47, wherein the method is a computer-implemented method.

49. A computer program product comprising instructions which, when the program is executed by a computer, cause the computer to carry out the method of any one of items 41-48.

50. A computer-aided detection (CAD) system for diagnosing an ovarian tumor of a patient using ultrasound image data of said patient, the system comprising:

- a data reception module arranged to receive the ultrasound image data;
- a lesion diagnosis module comprising a computer-readable medium having stored thereon a multi-class neural network for diagnosing ovarian tumors using ultrasound images;
- a malignancy scoring model, configured to calculate a malignancy score of the ultrasound image data.

51. The system according to item 50, wherein the multi-class neural network has been trained according to any one of items 1-38.

52. The system according to any one of items 50-51, wherein the system is arranged to carry out the method of any one of items 41-48.

53. The system according to any one of items 50-52, wherein the system comprises an integration interface arranged to facilitate data exchange and result sharing with electronic health records (EHR) systems and/or diagnostic workstations;

54. The system according to any one of items 50-53, wherein the system comprises a user interface configured for providing healthcare professionals with interactive tools for review of a case and decision support, such as wherein the user interface is adaptable to clinical workflows.

55. The system according to any one of claims 50-54, wherein the system comprises a ROI detector module arranged to annotate regions-of-interest (ROI) in the images.

56. The system according to any one of items 50-54, wherein the system comprises an image preprocessing unit configured to preprocess the ultrasound image data, such as wherein the image preprocessing unit is configured to process said image data by remove image artifacts and/or perform standardization of images, such as image size and/or quality.

57. The system according to any one of items 50-56, wherein the system comprises an ultrasound device, arranged to obtain ultrasound images of a patient, and to provide the obtained ultrasound images to the data reception module.

**Claims**

1. A method for training a multi-class neural network for aiding in diagnosing ovarian tumors using ultrasound images, the method comprising:

   a. obtaining ultrasound image data from a plurality of diagnostic centers, each center providing images of both malignant and benign ovarian tumors, the image data comprising a plurality of sets, wherein each set comprises image data of an ultrasound examination of an individual patient;
   b. annotating each image of the image data, comprising labeling each image of the image data with histological diagnoses organized into a pre-defined hierarchical ontology;
   c. training a series of initial neural networks to perform multi-class classification wherein said training comprises a leave-one-out cross validation, wherein, for each round of said cross validation, a different center is used as hold-out test data, and a new initial neural network is trained using training data that is derived from the remaining image data;
   d. evaluating the performance of each initial neural network of the series of initial neural networks;
   e. identifying an optimal set of hyperparameters that yield the most consistent performance across all hold-out centers, based on the performance and the hyperparameters of each initial neural network; and
   f. training a final neural network using the optimal set of hyperparameters.

2. The method according to claim 1, wherein the hierarchical ontology comprises a plurality of levels of granularity.

3. The method according to any one of the preceding items, wherein the image data comprises, malignant and benign cases of each center, and wherein the ratio of malignant to benign cases, for each center, is in the range from 1:0.8 to 1:1.2.

4. The method according to any one of the preceding claims, wherein each set comprises a mixture of grayscale and doppler ultrasound images.

5. The method according to any one of the preceding claims, wherein the step of evaluating comprises using each hold-out test data to evaluate the performance of the respective initial neural network, and calculating, for each initial neural network, at least one performance metrics.

6. The method according to any one of the preceding claims, wherein the step of identifying comprises identifying the optimal set of hyperparameters that yields the highest average performance and least variance across all hold-out centers.

7. The method according to any one of the preceding claims, wherein each neural network is trained using multi-class focal loss.

8. The method according to any one of the preceding claims, wherein the training data is derived from the image data by hierarchical sampling.

9. The method according to claim 8, wherein the hierarchical sampling is arranged such that the training data comprises images of each category in terms of ovarian lesion classification, imaging modality, device type, and the presence of calipers.

10. The method according to any one of claims 8-9, wherein the hierarchical sampling comprises use of a sampling weight for each of said category, and wherein said sampling weight is determined by the inverse of the frequency of each category.

11. A method of determining a malignancy score of an ovarian tumor, the method comprising:

   a) obtaining a set of ultrasound images of the ovarian tumor;
   b) providing the set of ultrasound images to a trained multi-class neural network that has been trained according to any one of claims 1-10, thereby determining the malignancy score of the ovarian tumor.

12. The method according to claim 11, wherein the step of providing comprises obtaining probability estimates, for each diagnostic classification of a hierarchical ontology, from the trained multi-class neural network.

13. The method according to any one of claims 11-12, wherein the method further comprises a step of computing the risk of malignancy for each ultrasound image of the set, by summing the probability estimates attributed to malignant sub-diagnosis groups.

14. A computer-aided detection (CAD) system for diagnosing an ovarian tumor of a patient using ultrasound image data of said patient, the system comprising:

   • a data reception module arranged to receive the ultrasound image data;
   • a lesion diagnosis module comprising a computer-readable medium having stored thereon a multi-class neural network for diagnosing ovarian tumors using ultrasound images, wherein the multi-class neural network has been trained according to any one of claims 1-10;
   • a malignancy scoring model, configured to calculate a malignancy score of the ultrasound image data.

15. The system according to claim 14, wherein the system comprises an ultrasound device, arranged to obtain ultrasound images of a patient, and to provide the obtained ultrasound images to the data reception module.

FIG.1

FIG.2

FIG.3

FIG.4

FIG.5

FIG.6A

FIG.6B

FIG.7

FIG.8

FIG.9A

FIG.9B

FIG.10

FIG.11

FIG.12A

## Junior Confidence

FIG.12B

Europäisches Patentamt

European Patent Office

Office européen des brevets

**EUROPEAN SEARCH REPORT**

**Application Number**

EP 23 22 0765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2023/242805 A1 (SYNDIAG S R L [IT]) 21 December 2023 (2023-12-21) | 1-6,8, 11,12, 14,15 | INV. G06T7/00 |
| A | * abstract; figures 1,7-8 * <br> * page 3, lines 4-12 * <br> * pages 14-18 * <br> * page 22, lines 14-18 * <br> ----- | 7,9,10, 13 | |
| Y | KNIGHT JESSE ET AL: "Voxel-Wise Logistic Regression and Leave-One-Source-Out Cross Validation for white matter hyperintensity segmentation", MAGNETIC RESONANCE IMAGING, ELSEVIER SCIENCE, TARRYTOWN, NY, US, vol. 54, 19 June 2018 (2018-06-19), pages 119-136, XP085499039, ISSN: 0730-725X, DOI: 10.1016/J.MRI.2018.06.009 | 1-6,8, 11,12, 14,15 | |
| A | * abstract; figure 6; table 4 * <br> * sections 3.1-3.2 * <br> * section 3.3.1 * <br> * section 4.2.4 * <br> ----- | 7,9,10, 13 | TECHNICAL FIELDS SEARCHED (IPC) <br><br> G06T |
| A | BEN VAN CALSTER ET AL: "Classifying ovarian tumors using Bayesian Multi-Layer Perceptrons and Automatic Relevance Determination: A multi-center study", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA, IEEE, PISCATAWAY, NJ, USA, 1 August 2006 (2006-08-01), pages 5342-5345, XP031187621, ISBN: 978-1-4244-0032-4 * abstract; figure 1; table 1 * * sections 3-4 * <br> ----- <br> -/-- | 1-15 | |

The present search report has been drawn up for all claims

| Place of search | Date of completion of the search | Examiner |
|---|---|---|
| Munich | 18 June 2024 | Fronthaler, Hartwig |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches
Patentamt

European
Patent Office

Office européen
des brevets

# EUROPEAN SEARCH REPORT

**Application Number**

EP 23 22 0765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SOPER DANIEL S: "Greed Is Good: Rapid Hyperparameter Optimization and Model Selection Using Greedy k-Fold Cross Validation", ELECTRONICS, vol. 10, no. 16, 16 August 2021 (2021-08-16), XP093173769, Basel, Switzerland ISSN: 2079-9292, DOI: 10.3390/electronics10161973 Retrieved from the Internet: URL:https://www.mdpi.com/2079-9292/10/16/1973> * abstract; figures 8,9 * * section 2.3 * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 18 June 2024 | Fronthaler, Hartwig |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 22 0765

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2024

| Patent document cited in search report | Publication date | Patent family member(s) | Publication date |
|---|---|---|---|
| WO 2023242805 A1 | 21-12-2023 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VASWANI et al.** *Attention is All You Need*, 2017
- **TOUVRON et al.** *Training Vision Transformers on Noisy Large-Scale Data*, 2020